# EUROPEAN PATENT APPLICATION

(11) **EP 4 227 423 A1**
(43) Date of publication of application: **16.08.2023**
(21) Application number: 22155928.9
(22) Date of filing: 09.02.2022
(51) Int. Cl.: C12Q 1/6886, A61K 35/00

(54) **MEANS AND METHODS FOR IDENTIFYING HIGH AFFINITY T-CELLS**

(71) Applicant: Technische Universität München, 80333 München (DE)
(72) Inventor: BUSCH, Dirk, 83727 Schliersee (DE); SCHOBER, Kilian, 91054 Erlangen (DE); PURCAREA, Anna, 81675 Munich (DE)
(74) Representative: Schiweck Weinzierl Koch Patentanwälte Partnerschaft mbB

(57) **Abstract**

The present invention relates to a method of identifying one or more T cells, as well as method of isolating one or more T cells. Further, the invention relates to a method of identifying one or more TCR, a nucleic acid molecule comprising a nucleic acid sequence encoding the TCR identified by the methods of the invention. The invention also relates to a method of generating one or more immune cells. The invention also relates to a host cell comprising a nucleic molecule encoding a TCR identified by the invention or generated by a method of the invention. The invention also relates to a pharmaceutical composition comprising a cell of the invention, or a cell of the invention for use in therapy. Finally, the invention also relates to a method of diagnosing cancer.

## Description

### TECHNICAL FIELD OF THE INVENTION

The present invention relates to a method of identifying one or more T cells, as well as method of isolating one or more T cells. Further, the invention relates to a method of identifying one or more TCR, a nucleic acid molecule comprising a nucleic acid sequence encoding the TCR identified by the methods of the invention. The invention also relates to a method of generating one or more immune cells. The invention also relates to a host cell comprising a nucleic molecule encoding a TCR identified by the invention or generated by a method of the invention. The invention also relates to a pharmaceutical composition comprising a cell of the invention, or a cell of the invention for use in therapy. Finally, the invention also relates to a method of diagnosing cancer.

### BACKGROUND OF THE INVENTION

Tumor antigen-specific T cells are pivotal for the control of cancer (Schreiber, R. D., Old, L. J. & Smyth, M. J. Cancer immunoediting: integrating immunity's roles in cancer suppression and promotion. Science 331, 1565-70 (2011)). This knowledge has been therapeutically harnessed by the use of tumor-infiltrating lymphocytes (TILs), donor lymphocyte infusions or, more recently, T cells with a transgenic tumor-specific T cell receptor (Stadtmauer, E. A. et al. Long-term safety and activity of NY-ESO-1 SPEAR T cells after autologous stem cell transplant for myeloma. Blood Adv. 3, 2022-2034 (2019)). The ability to engineer T cells with a defined T cell receptor (TCR) is now possible in a highly precise fashion (Schober, K. et al. Orthotopic replacement of T-cell receptor α- and β-chains with preservation of near-physiological T-cell function. Nat. Biomed. Eng. 3, 974-984 (2019)). This opens up new avenues for personalized medicine, but in turn also illustrates the relevance of understanding how tumor-specific T cells are programmed by their TCR. A central question is how tumor antigen-specific T cells with protective (high functionality) TCRs can be identified at different tissue sites. Deeper insights into the TCR driven spatiotemporal fate of T cells will thereby make the behavior of T cell products for adoptive cell therapy and the use of checkpoint inhibitory therapy more predictable, and guide *de novo* identification of protective TCRs.

During tumor disease, the composition of the T cell receptor (TCR) repertoire of tumor-specific CD8+ T cells changes in space and over time. Although TCR affinity is assumed to be a major determinant of the spatiotemporal fate and protective capacity of tumor-specific T cells, experimental evidence for this is still scarce. The effects of TCR affinity on the spatiotemporal fate of T cells and on their protective capacity against tumors remain poorly understood. Importantly, previous studies concentrated on tumor antigen specificity, and did not take the affinity of a given tumor-specific TCR into account. This is particularly noteworthy since -beyond mere antigen specificity - TCR affinity to its ligand is widely assumed to be one of the most important determinants of a T cell response (Tscharke, D. C., Croft, N. P., Doherty, P. C. & La Gruta, N. L. Sizing up the key determinants of the CD8(+) T cell response. Nat. Rev. Immunol. 15, 705-16 (2015)). Most studies find that high-affinity T cells are better at tumor infiltration than low-affinity T cells. Perhaps for this reason, high-affinity tumor-specific T cells have also been reported to preferentially undergo T cell exhaustion although the opposite has likewise been shown. Furthermore, it is unclear whether the prognostic value and the expression levels of PD-1 on tumor antigen-specific T cells (within or outside the tumor) depends on TCR affinity. A major technical difficulty to address these questions is the lack of methods that can identify TCR sequences and characterize their affinity at the same time. Many of the studies that did investigate the role of affinity in anti-tumor T cell immunity used human antigen mouse models examined responses against a self-antigen followed T cell fate after monoclonal transfers, adoptively transferred high cell numbers and/or included immunization schemes in addition to cell transfers. Such approaches are highly valuable for translational research on TCR candidates targeting human tumor antigens, but are limited in reflecting physiological anti-tumor T cell immunity since antigen-specific T cell populations develop from small numbers of precursor cells (Alanio, C., Lemaitre, F., Law, H. K. W., Hasan, M. & Albert, M. L. Enumeration of human antigen-specific naive CD8+ T cells reveals conserved precursor frequencies. Blood 115, 3718-25 (2010)) and are polyclonal (Sims, J. S. et al. Diversity and divergence of the glioma-infiltrating T-cell receptor repertoire. Proc. Natl. Acad. Sci. 201601012 (2016) doi:10.1073/pnas.1601012113). Tracking TCR affinity-dependent T cell fate is therefore challenging, which renders surrogate markers for TCR affinity a particular relevance.

The technical problem underlying the present application is to comply with this need. The solution to said technical problem is the provision of means and methods as reflected in the claims, described herein, illustrated in the Figures and exemplified in the Examples of the present application.

### SUMMARY OF THE INVENTION

The present invention relates to a method of identifying one or more T cells comprising: a) determining in a peripheral blood sample derived from a subject the expression level of PD-1 in a population of T cells; and b) identifying one or more T cells that have an expression level of PD-1 that is above the median expression of PD-1 in the population of T cells.

The invention also relates to a method of identifying one or more T cells comprising: a) determining in a peripheral blood sample the mRNA expression level of one or more markers selected from the group consisting of GZMB, CCL3, UNC13D, BTG1, BTG2, CLU, IRS1, and BCL2L in a population of T cells; and b) identifying one or more T cells that fulfill at least one of the following features: increased GZMB expression, increased CCL3 expression, increased UNC13D expression, increased BTG1 expression, increased BTG2 expression, increased CLU expression, increased IRS1 expression, and/or increased BCL2L expression.

The present invention further envisages a method of isolating one or more T cell, comprising isolating one or more T cells identified according to a method of the invention.

The present invention also relates to a method of identifying one or more TCR comprising determining the sequence of the TCR of the one or more T cells identified by a method of the invention.

The present invention further envisages a nucleic acid molecule comprising a nucleic acid sequence encoding the TCR identified by a method of the invention or encoding a TCR comprised in a T cell identified by a method of the invention.

The present invention relates to a method of generating one or more immune cells comprising introducing one or more nucleic acid molecules encoding one or more TCRs or antigen-binding fragments thereof, identified by a method of the invention into one or more host cells.

The present invention relates to a host cell comprising a nucleic acid molecule of the invention or generated by a method of the invention.

The present invention envisages a pharmaceutical composition comprising a T cell identified by a method of the invention or isolated by a method of the invention, an immune cell generated by a method of the invention, or a host cell of the invention.

The present invention also relates to a T cell identified by a method of the invention or isolated by a method of the invention, an immune cell generated by a method of the invention, or a host cell of the invention for use in therapy.

The present invention also relates to a method of diagnosing cancer in a subject comprising measuring the PD-1 expression level in a peripheral blood sample obtained from the subject, wherein an elevated PD-1 expression level as compared to a reference blood sample from a healthy control indicates the presence of T cells having high affinity TCRs.

### BRIEF DESCRIPTION OF THE DRAWINGS

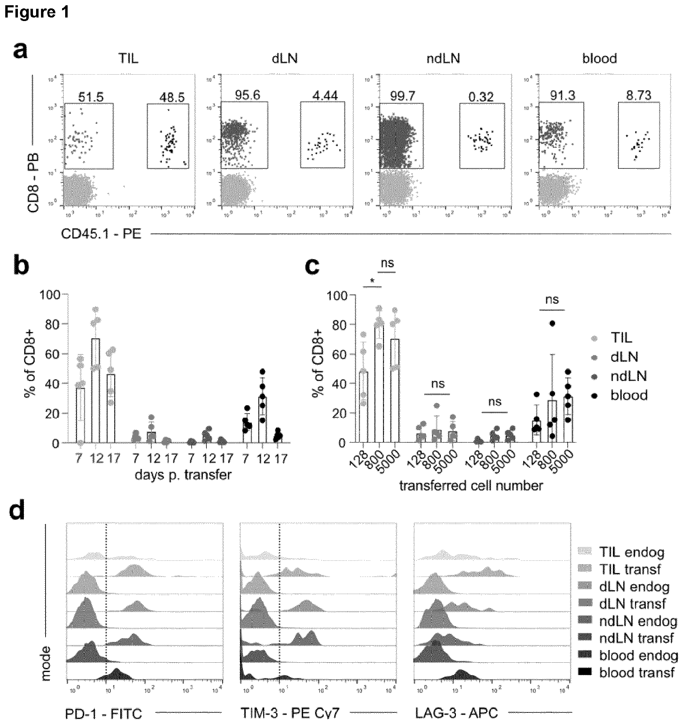

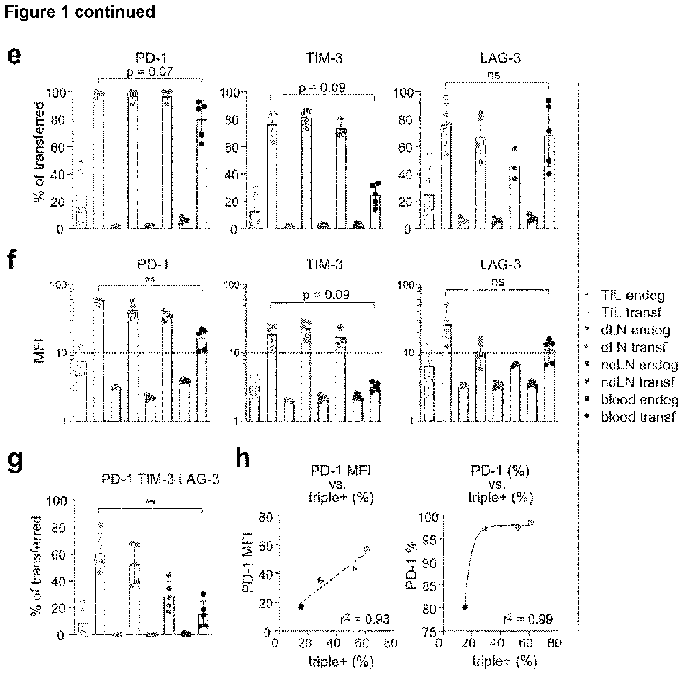

**Figure 1****. PD-1 expression levels correlate with global upregulation of co-inhibitory receptors and increase from peripheral blood to tumor.** (a) Flow cytometric analysis of transferred (CD45.1+) CD8+ T cells among tumor infiltrating lymphocytes (TIL), in draining lymph nodes (dLN), in non-draining lymph nodes (ndLN) and in peripheral blood, pregated on living, CD19- lymphocytes. Mice were irradiated with 5 Gy TBI (total body irradiation) and given 5.000 OT-I cells i.p. followed by injection of 106 MC38 OVA cells s.c. the next day. (b) Quantification from (a), with percentage of transferred cells for different time points, color coded for organs of origin. (c) Experimental setup as in (a) except that initially the shown numbers of OT-I cells were transferred and data are shown for day 12 only. Statistical testing by one-way ANOVA (*(TIL) or ns (dLN, ndLN, blood)) followed by Tukey's multiple comparisons test with n = 5 mice (results of which are indicated in graph). (d) Histograms showing representative flow cytometry analyses of PD-1, TIM-3 and LAG-3 phenotypes of transferred and endogenous CD8+ cells, quantified in (e) by percentage of PD-1, TIM-3 and LAG-3 positive cells and in (f) by mean fluorescence intensity (MFI). Dashed lines in (d) and (f) illustrate cut-off for positive populations based on negative controls. Statistical testing by Kruskal-wallis (* (PD-1%, LAG-3 MFI), ** (TIM-3 MFI), *** (TIM-3%),**** (PD-1 MFI) or ns (LAG-3%)) followed by Dunn's multiple comparisons test with n = 5 mice. (g) Percentage of PD-1/TIM-3/LAG-3 triple positive cells. Statistical testing by Kruskal-Wallis test (**) followed by Dunn's multiple comparisons test (results of which are indicated in graph) with n = 5 mice. (h) Correlation of PD-1 MFI and percentage of PD-1+ cells of transferred cells with fraction of triple positive cells. Fitted with linear fit for mean of n = 5 mice for each organ. ns, not significant. * p value < 0.05, ** p value < 0.01, *** p value < 0.001, **** p value < 0.0001.

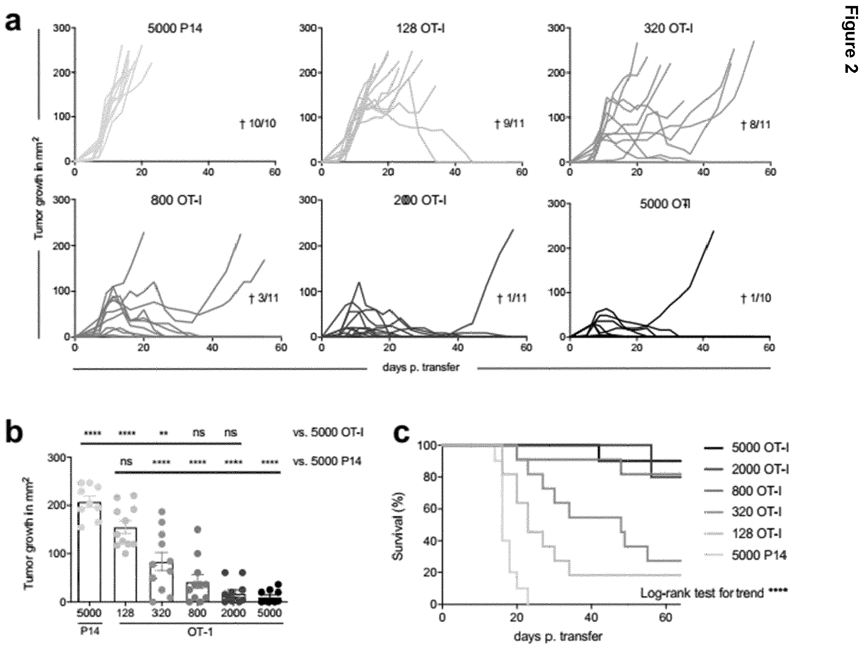

**Figure 2****. Ultra-low numbers of tumor-specific T cells can mediate protection. (a)** Tumor growth of mice that were irradiated with 5 Gy TBI (total body irradiation) and given OT-I cells i.p. followed by injection of 106 MC38 OVA cells s.c. the next day. Administration of either 128, 320, 800, 2000 or 5000 OT-I cells, negative control was performed with 5000 P14 cells. **(b)** Mean tumor growth (±SEM) on day 16 post T cell transfer, statistical testing by one-way ANOVA (****) followed by Tukey's multiple comparisons test (results of which are indicated in graph). **(c)** Survival curves (n = 11 mice). Statistical testing by log-rank test for trend. Data are representative of two independent experiments with n = 5-6 mice. ns, not significant. ** p value < 0.01, **** p value < 0.0001.

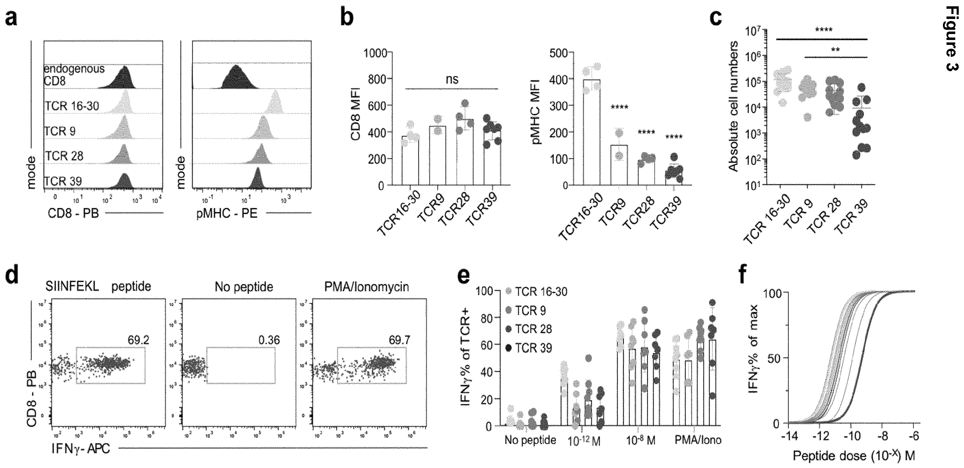

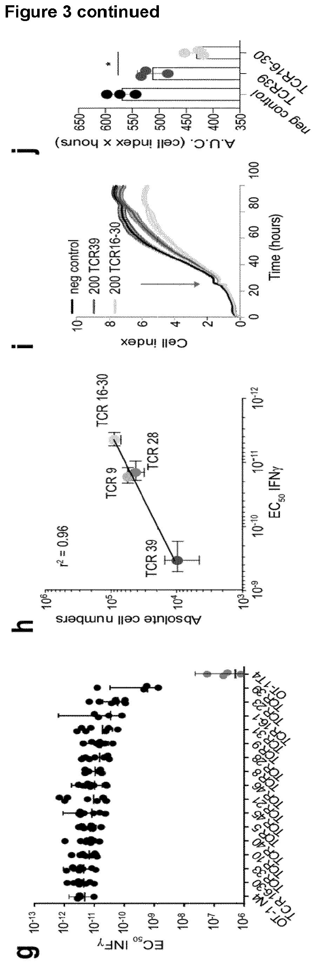

**Figure 3****. A SIINFEKL-specific TCR library provides TCRs with distinct functional profiles. (a)** Histograms of representative flow cytometry analyses of CD8 and H2Kb/OVA257-264 pMHC-multimer expression of retrogenic TCRs with different structural avidity for the SIINFEKL peptide compared to endogenous CD8+ T cells. **(b)** Quantification of MFI from (a). Statistical testing by one-way ANOVA (****(pMHC MFI) or ns (CD8 MFI)) followed by Tukey's multiple comparisons test (results of which are indicated in graph), n = 2-9 mice. **(c)** *In vivo* recruitment of retrogenic TCRs, quantified by absolute numbers of transferred cells in spleens on day 12 after i.p. transfer of 100 T cells retrogenic for each TCR as well as i.v. infection with 5.000 CFU of *L.m.* OVA. Statistical testing by Kruskal-Wallis test (****) followed by Dunn's multiple comparisons test (results of which are indicated in graph) with n = 12 mice from two independent experiments. **(d)** Flow cytometry analysis of interferon-γ (IFNγ) release after *ex vivo* peptide stimulation of cells from (c) and subsequent intracellular cytokine staining (ICCS). Splenocytes were stimulated with SIINFEKL peptide, no peptide or PMA/lonomycin. Pregating on CD8+ cells, numbers indicate percentages. **(e)** Frequencies of IFNγ+ cells after stimulation with no peptide, a peptide concentration of 10-12 M, of 10-6 M or with PMA/lonomycin. **(f)** IFNγ release after stimulation with 10-14 M, 10-12 M, 10-11 M, 10-10 M, 10-9 M, 10-8 M, 10-6 M SIINFEKL peptide was fitted by non-linear regression curves. **(g)** Quantification of peptide concentrations needed for half maximal IFNγ release (EC50) for a panel of 15 retrogenic TCRs and OT-I after stimulation with SIINFEKL as well as for OT-I after stimulation with the SIITFEKL altered peptide ligand. n = 4-9 biological replicates from 2-3 independent experiments **(h)** Correlation of absolute cell numbers of transferred cells from (c) and their EC50 value for IFNγ production from (g), fitted with non-linear fit (log-log). **(i)** *In vitro* killing capacity of retrogenic TCRs. Mice were injected with at least 300 retrogenic TCR+ T cells and infected with 5.000 CFU of *L.m.* OVA. Mice were sacrificed on day 8 post infection and their spleens harvested. 200 flow cytometry-sorted CD8+ TCR+ cells were incubated with PancOVA cells and killing was monitored via changes in cell index (i). **(j)** Killing capacity was quantified via the area under the curve (AUC) for no cells, 200 TCR 39 and 200 TCR 16-30 cells. Statistical testing by one-way ANOVA (**) followed by Tukey's multiple comparisons test (results of which are indicated in graph) with n = 3 technical replicates. ns, not significant. * p value < 0.05, ** p value < 0.01, **** p value < 0.0001.

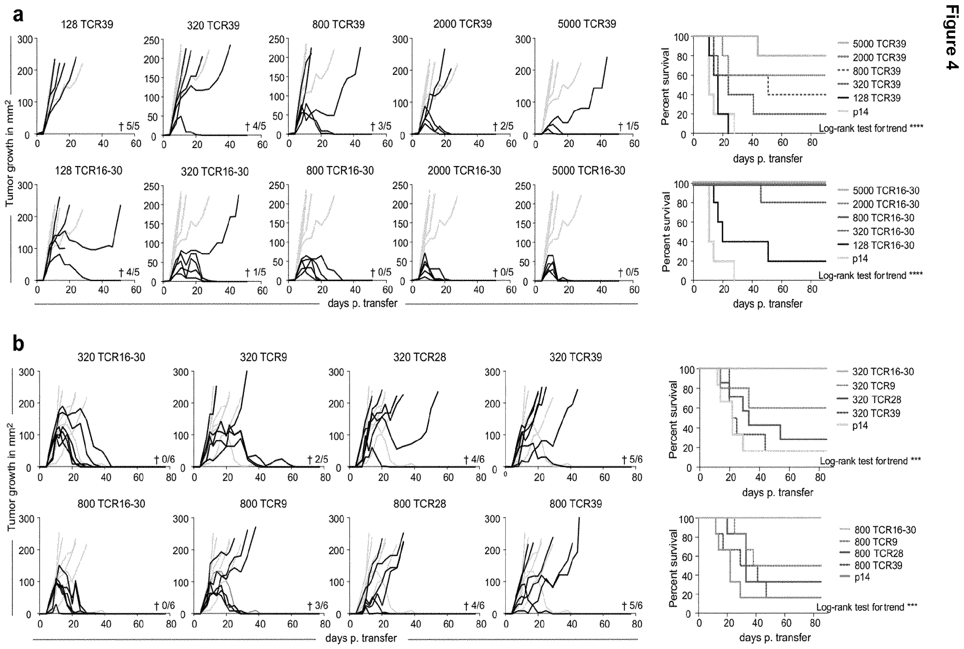

**Figure 4****. Differential tumor protection in vivo through TCRs with distinct affinities.** Mice were irradiated with 5 Gy TBI (total body irradiation) and given TCR+ cells i.p., followed by injection of 106 cells MC38 OVA s.c. the next day. Administration of **(a)** 128, 320, 800, 2000 or 5000 either TCR16-30 or TCR39 cells, negative control with 5000 P14 cells, **(b)** 320 or 800 cells of either TCR9, TCR28, TCR16-30 or TCR39, negative control with 800 P14 cells. Shown are tumor growth and corresponding survival curves. Statistical testing by log rank (Mantel-cox) for trend with n = 5-6 mice.

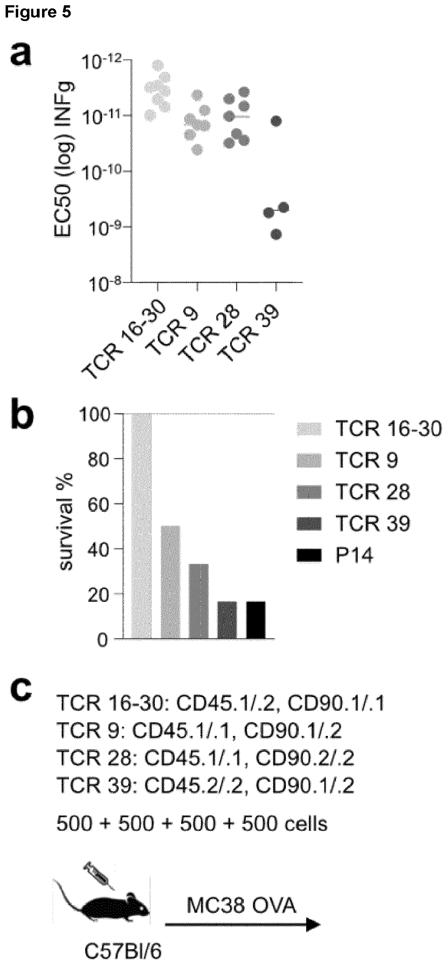

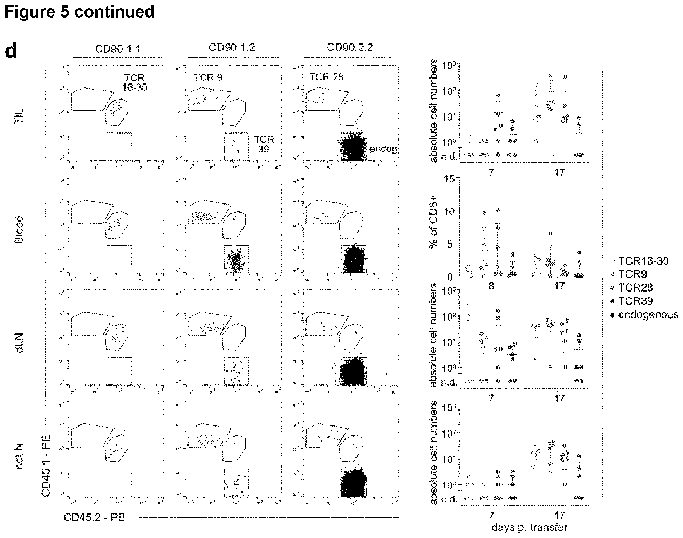

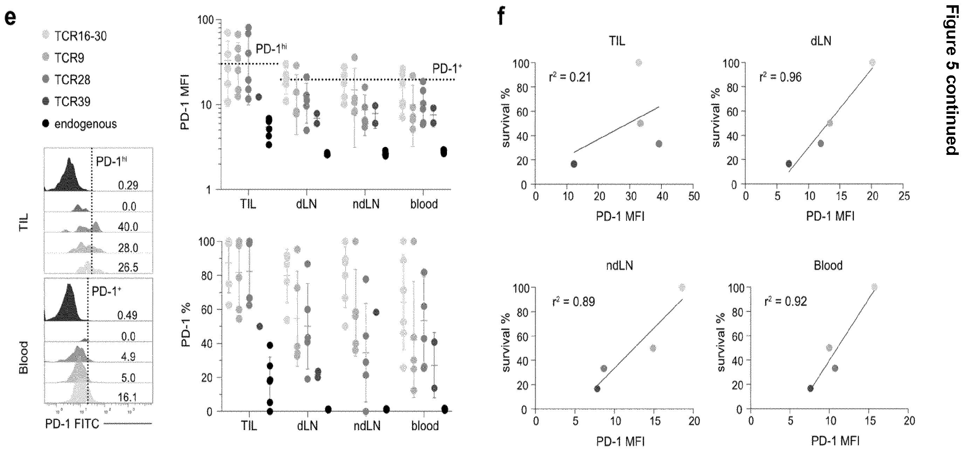

**Figure 5****. In polyclonal populations TCR protective capacity correlates with PD-1 expression outside the tumor. (a)** Comparison of EC50 values for IFNγ production for selected retrogenic TCRs from Fig. 3g. **(b)** Survival in percentage from Fig. 4b (lower panel, after transfer of 800 TCR+ T cells). **(c)** Scheme of experimental setup with simultaneous transfer of four different retrogenic TCRs barcoded via congenic markers CD45.1/.2 and CD90.1/.2 (500 cells per TCR amounting to 2000 cells overall) after TBI. Injection of 106 cells MC38 OVA the day after. **(d)** Flow cytometric analysis of transferred and endogenous T cells for different organs, pregated on CD19- CD8+ cells shown for day 17. Identification of specific TCR+ transferred T cells via unique combinations of the congenic markers (left). Absolute cell numbers for TIL and LNs as well as percentage of CD8+ transferred T cells for peripheral blood, depicted for each TCR on day 7 (analysis for peripheral blood was performed on day 8) and 17 (right). n = 5 mice. **(e)** Percentage of PD-1+ (left) and PD-1 MFI (middle and right) of transferred TCR+ T cells in different organs compared to the endogenous CD8+ T cell population. Data are shown for day 17 of experiment from (c) and (d). Based on the endogenous CD8+ T cell population, MFI cut-offs for PD-1+ (outside the tumor) and PD-1hi (in the tumor) were defined, as indicated by dashed lines. n = 5 mice (subpopulations were excluded for which less than 10 cells could be used for MFI analysis, which only applied for TCR39 subpopulations) **(f)** Correlation of survival (b) and mean PD-1 MFI (e) for the different retrogenic TCRs, fitting by nonlinear regression. Data are representative of two independent experiments. Statistical testing in (a) and (e) by one-way ANOVA. * p value < 0.05, ** p value < 0.01, **** p value < 0.0001.

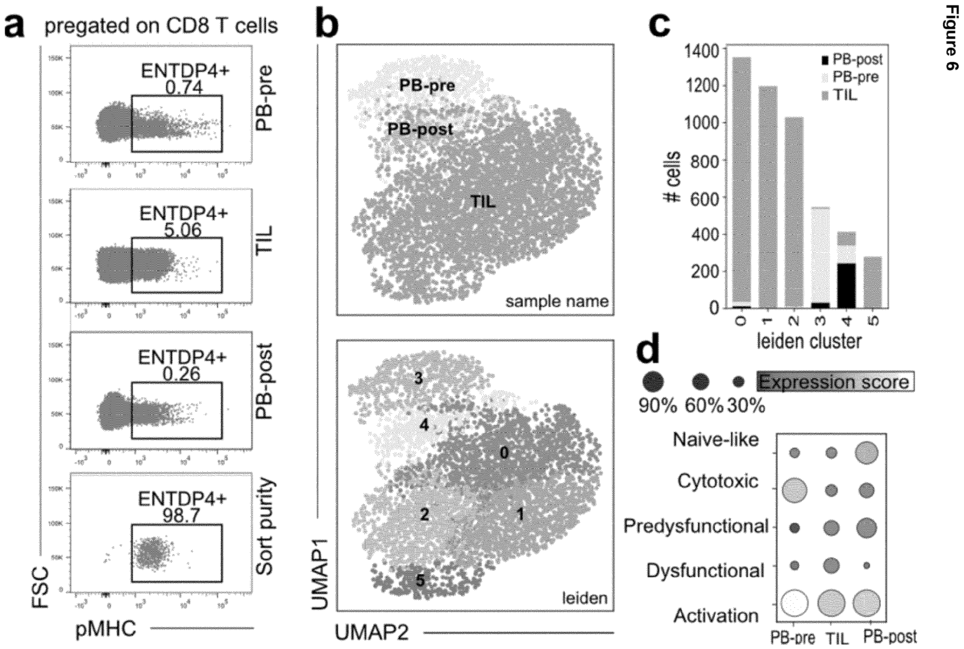

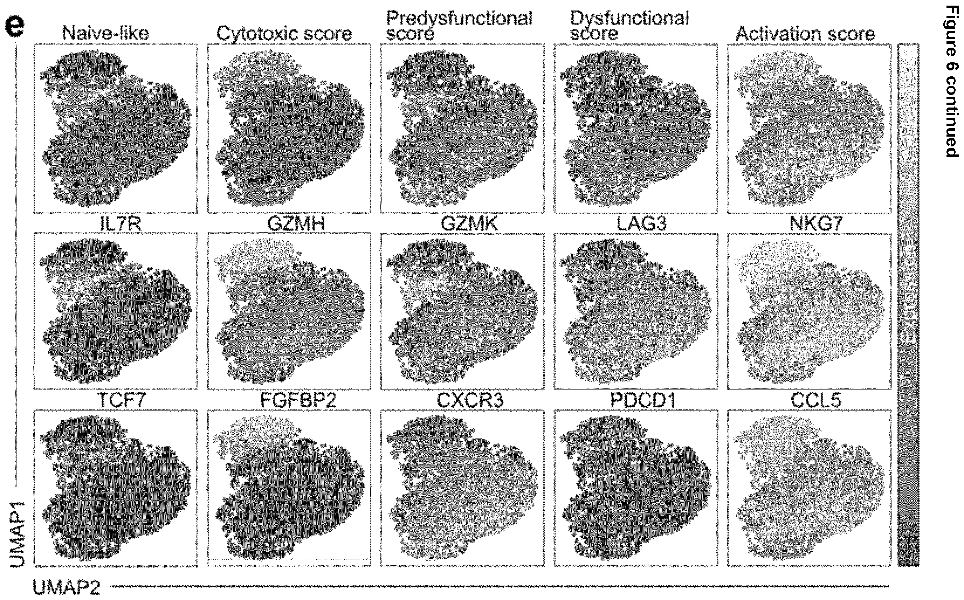

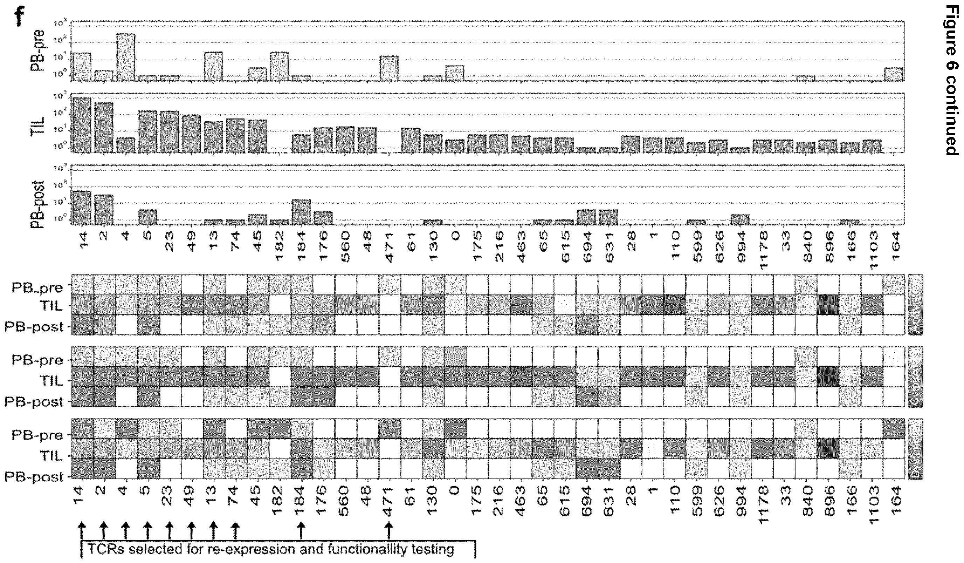

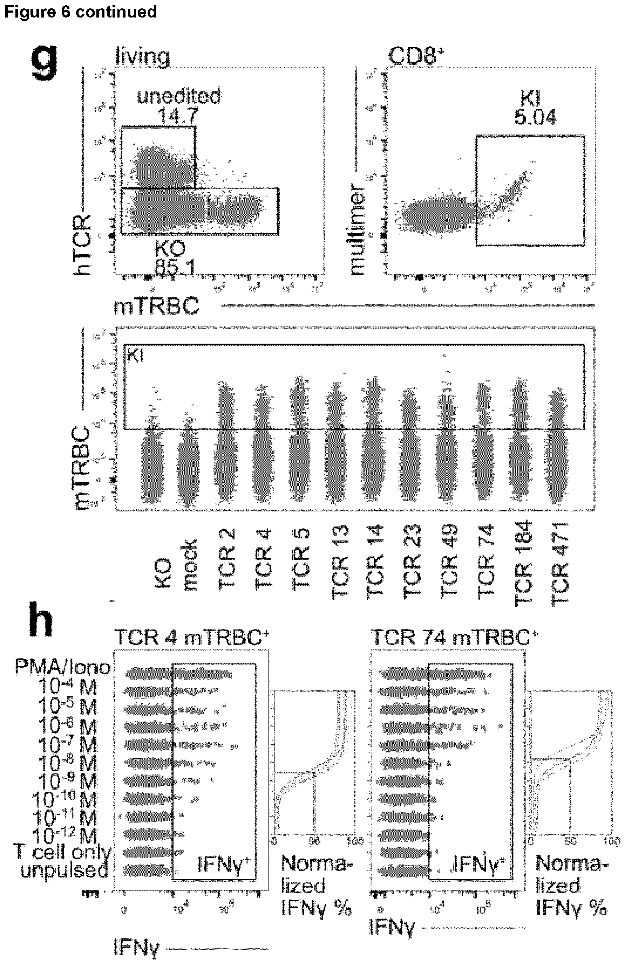

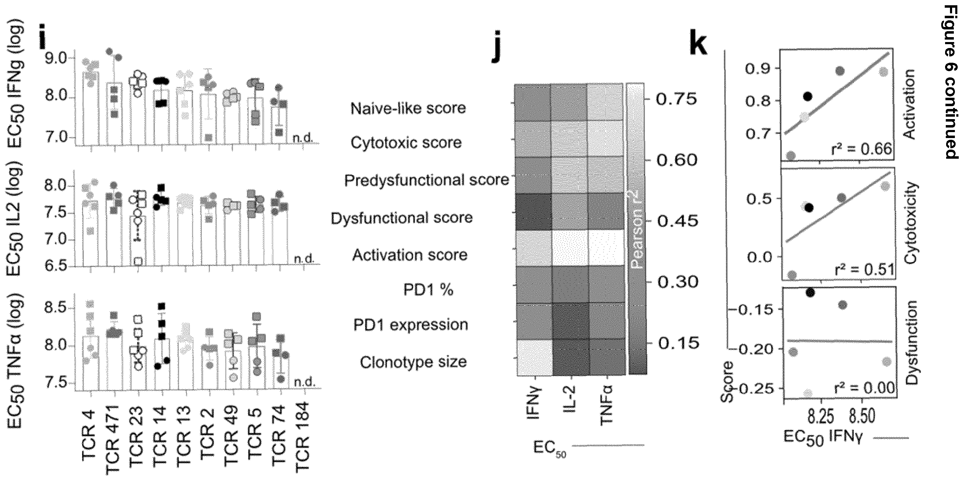

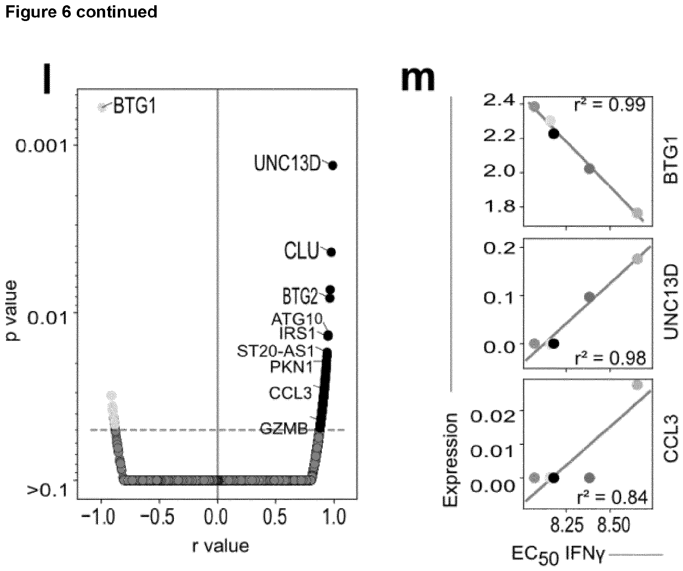

**Figure 6****. Phenotypic correlates of high-functionality TCRs among human neoantigen-specific T cells in peripheral blood.** (**a**) ENTDP4-multimer+ CD8+ T cells were sorted from a patient before TIL treatment (PB-pre), after TIL treatment (PB-post) and from the infusion product (TIL). Numbers indicate percentages (**b**) UMAPs depict neighborhood embedding based on the transcriptomic data of 4775 single cells. The color depicts sample source (top) or leiden cluster (bottom) (**c**) Distribution of cells across leiden clusters. The color depicts sample source (**d**) Gene set scores are shown as heat map for each sample source. The size of the dots represents the relative number of cells reaching the score (**e**) Expression scores (top row) and expression of two representative genes from the gene set (middle and lower row) are depicted for individual cells on the UMAP (**f**) The size per ample source of all individual clonotypes that consist of at least three cells is depicted as bar graph (upper panel) and their corresponding scores as heat map (lower panel). Selected clonotypes for functional validation are marked by arrows. Squares are white if no cells are present the sample source and gray if less than three cells were detected. (**g**) Knock-in efficiency (top) and expression profiles (bottom) of T cell after CRISPR/Cas9 engineering are depicted). Numbers indicate percentages (**h**) Representative analysis of intracellular cytokine staining analysis of the edited cells. (**i**) EC50 values for the production of IFNγ, IL-2 and TNFα. Circle or square symbols represent values from two independent experiments. (**j**) Heat map depicting the r² value (correlation coefficient) for a linear regression between the scored gene sets in the blood of the patient before TIL infusion and the EC50 values after re-expression. Significant correlations (p value < 0.05) were detected for activation score vs EC50 IL-2/EC50 TNFα, size vs EC50 IFNγ and TRAC expression vs EC50 IL-2. (**k**) Exemplary plots for linear regression analyses. TCR color code according to (i). (**I**) Volcano Plot depicts the correlation between IFNγ EC50 values and the gene expression for all genes across the dataset. Significantly correlated genes (p < 0.05, indicated by dashed line) are depicted in black. (**m**) Exemplary plots for linear regression analyses between mean gene expression per clonotype and IFNγ EC50 as depicted in (I). TCR color code according to (i). ns, not significant. * p value < 0.05, ** p value < 0.01, *** p value < 0.001, **** p value < 0.0001. Mean ± SD is depicted for EC50 values in (i) and the mean was used for correlation analysis in (j-m). Two-sided p-values for a hypothesis test (null hypothesis: slope is zero) have been calculated in (j-m), using Wald Test with t-distribution of the test statistic.

### DETAILED DESCRIPTION OF THE INVENTION

The inventors here set out to develop methods to isolate T cell preferably having high affinity TCRs for tumor antigens. Such high affinity TCR are believed to be correlated with high tumor protection. As used herein, the term "TCR" refers to a T cell receptor. The T-cell receptor (TCR) is a protein complex found on the surface of T cells, or T lymphocytes, that is responsible for recognizing fragments of antigen as peptides bound to major histocompatibility complex (MHC) molecules. The TCR is composed of two different protein chains (that is, it is a hetero dimer). In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). This ratio changes during ontogeny and in diseased states (such as leukemia). It also differs between species. Orthologues of the 4 loci have been mapped in various species. Each locus can produce a variety of polypeptides with constant and variable regions. When the TCR engages with antigenic peptide and MHC (peptide/MHC), the T lymphocyte is activated through signal transduction

The inventors surprisingly found that high PD-1 expression levels within T cells populations found in peripheral blood samples, and general signatures of recent T cell activation within T cells populations found in peripheral blood samples can therefore serve as a surrogate marker for highly protective TCRs (i.e TCR with high affinity and displaying high tumor protection, as defined herein). This finding is important since it allows to correlate the phenotype of T cell clones in easily accessible compartments (e.g. peripheral blood) with actual TCR avidity/functionality. Previous studies have not tracked TCRs with different affinity against the same epitope as part of one polyclonal population reacting against a tumor. That TCR affinity correlates with PD-1 expression levels outside the tumor is surprising because based on previous studies, it could be assumed that all antigen specific T cells (irrespective of affinity) would become PD-1 positive upon antigen encounter and not be different in terms of expression PD-1 levels (as is the case in the tumor, only on a higher absolute expression level). Therefore, the finding of the present invention is of importance because it allows to identify T cells or TCRs with high functionality based on a simple biomarker in a compartment that is easily accessible, such as peripheral blood.

Peripheral blood sample is a widely accessible source in patients, which can be derived with non-invasive procedures. In particular, while obtaining T cells directly from tumor tissue (i.e.Tumor infiltrating Lympocytes or TILs) requires invasive intervention (e.g. biopsy), using peripheral blood as sample allows the use of non-invasive techniques which are both easy to perform, and inexpensive. Therefore, in the context of the invention peripheral blood represents an easily obtainable biomaterial. The term "easily obtainable biomaterial" can interchangeably be used with the terms "easily accessible biomaterial" or "easily available biomaterial". "Easily obtainable biomaterial" means in this respect that said biomaterial can be taken or achieved from a subject without the use of risky invasive methodologies or interventions, such as biopsies, in particular organ biopsies. Hence, such "easily obtainable biomaterial" can be quickly derived from a subject or patient. "Obtained or obtainable" means in this respect that the biomaterial is derived from said subject using any methods or means known to the person skilled in the art that allow to take a sample from said subject. Preferably, for obtaining whole blood from a subject, tools like syringes or lancets are applied.

When referring to PD-1 expression levels, extratumoral T cell populations are said to have lower levels of PD-1 expression in comparison with the levels of PD-1 expression levels found inside a solid tumor (in particular, in tumor infiltrating lymphocytes (TIL)). Compared to expression levels of PD-1 in TIL, PD-1 protein levels which are found in the overall population of extratumoral T cells (i.e T cells found outside a solid tumor, in particular in draining Lymph nodes (dLN), non-draining Lymph nodes (ndLN), and peripheral blood samples may be considered as intermediate, wherein peripheral blood samples comprise T cells with the lowest PD-1 expression when compared to dLN, ndLN and TILs). Accordingly, a T cell from peripheral blood that has high PD-1 expression as compared to other T cells from peripheral blood, may only have an intermediate PD-1 expression as compared to TILs.

Accordingly, the present invention relates to a method of identifying one or more T cells comprising: a) determining in a peripheral blood sample derived from a subject the expression level of PD-1 in a population of T cells; and b) identifying one or more T cells that have an expression level of PD-1 that is above the median expression of PD-1 in the population of T cells found in said peripheral blood sample. PD-1 (Programmed cell death protein 1, also known as CD279 (cluster of differentiation 279), is a cell surface receptor that belongs to the immunoglobulin superfamily and is expressed on T cells and pro-B cells. PD-1 binds two ligands, PD-L1 and PD-L2. PD-1 has a role in regulating the immune system's response to the cells of the human body by down-regulating the immune system and promoting self-tolerance by suppressing T cell inflammatory activity. This prevents autoimmune diseases, but it can also prevent the immune system from killing cancer cells. PD-1 is an immune checkpoint and guards against autoimmunity through two mechanisms. First, it promotes apoptosis (programmed cell death) of antigen-specific T-cells in lymph nodes. Second, it reduces apoptosis in regulatory T cells (anti-inflammatory, suppressive T cells). The PD-1 protein in humans is encoded by the PDCD1 gene.

The term "identifying" when referred to the one or more T cells according to the invention may therefore refer to the selection of T-cells via any method that can be used to measure (or determine) the expression level of PD-1 in a population of T-cells. In particular, according to the disclosure the selected T-cells are characterized by having high affinity TCRs (defined elsewhere herein). The term "determining" as used herein, when referring to the expression levels of PD-1 may comprise any technique useful to determine the expression levels of PD-1 (protein) in a population of T-cells, for example, expression levels of PD-1 may be determined by flow cytometry, as described in details in the examples section. In the context of the present invention, the expression level of PD-1 therefore preferably refers to the amount of PD-1 protein present in the T cells of the T cell population. Accordingly, the amount of PD-1 protein (or protein levels) in the T cells of the invention can be measured by methods known to the skilled artisan, in particular, the amount of PD-1 protein expressed by the cells of the invention is preferably measured using FACS. Various techniques to measure protein expression levels are known in the art, one of such techniques is Flow cytometry. Flow cytometry is a technique known to the skilled artisan, used to detect and measure physical and chemical characteristics of a population of cells or particles. In this process, a sample containing cells or particles is suspended in a fluid and injected into the flow cytometer instrument. The sample is focused to ideally flow one cell at a time through a laser beam, where the light scattered is characteristic to the cells and their components. Cells are often labelled with fluorescent markers so light is absorbed and then emitted in a band of wavelengths. Tens of thousands of cells can be quickly examined and the data gathered are processed by a computer. In particular, the inventors determined the expression levels of PD-1 by determining PD-1 MFI (mean fluorescence intensity) as described in Examples 1 and 5 and show in Figures 1 and 5. Mean Fluorescent Intensity (MFI), as known to the skilled artisan, is often used to compare expression of a target of interest (TOI) across samples/ cell populations in Flow cytometry. The MFI gives reliable information about expression levels/ presence of TOI within the experiment.

As discussed herein, the disclosed methods envisage that after determining the PD-1 expression levels (step a), one or more T cells having PD-1 expression levels above the median expression of PD-1 in the population of T cells derived from a peripheral blood sample are identified (i.e. selected). In particular, the selected one or more T cells are one or more T cells obtained from a peripheral blood sample that have an expression level of PD-1 that is the PD-1 expression level found within the about top 20%, preferably the PD-1 expression level found within the about top 10%, preferably the PD-1 expression level found within the about top 5%, preferably the PD-1 expression level found within in the about top 4% of the population of T cells obtained from said peripheral blood sample. As the inventors surprisingly found, in the population of PD-1 positive T cells found in blood sample, preferably the about top 20%, preferably the about top 10%, preferably the about top 5%, preferably the about top 4% PD-1 expressing cells are comprising T cells with high affinity TCR, defined elsewhere herein. In preferred embodiments, the one or more T cells identified (i.e. selected) are T-cells that are the top PD-1 expressing T cells in a peripheral blood sample, preferably, the about top 4% PD-1 expressing T-cells in a peripheral blood sample and, surprisingly, these T cells are also expressing high affinity TCRs (see Example 5 and Figure 6b). Therefore, within the T cell population found in peripheral blood sample, the T cells having the highest PD-1 expression level (in particular, a PD-1 expression level above the median expression levels within the T cell population, preferably the top 4% PD-1 expressing T-cells in a peripheral blood sample) are T cells having high affinity TCRs. The term "expression" when used herein can generally refer to mRNA expression or protein expression. In the context of PD-1 expression, the term expression preferably refers to protein expression. The inventors of the present application have surprisingly found that PD-1 protein expression has a stronger correlation with TCR affinity as compared to the correlation between PD-1 mRNA expression and TCR affinity.

In the context of the present invention, a method of identifying one or more T cells may envision that the PBMCs present in the peripheral blood sample are isolated, and subsequently used to prepare a single-cell suspension. Said T cells in single cell suspension and PD-1 are stained with fluorochrome-conjugated antibodies for flow cytometric analysis-potentially pMHC multimer staining may be included to detect antigen-specific T cells. The stained cells are subsequently analyzed by flow cytometry, and potentially by cell sorting to identify T cell receptor sequences subsequently (e.g. via single-cell RNA sequencing). Cell sorting and single cell RNA sequencing are techniques known to the skilled artisan.

According to the invention a method to identify one or more T cells may comprise a) determining in a peripheral blood sample the mRNA expression level of one or more markers, selected from the group consisting of GZMB, CCL3, UNC13D, BTG1, BTG2, CLU, IRS1, and BCL2L in a population of T cells; and b) identifying one or more T cells that fulfill at least one of the following features: increased GZMB expression, increased CCL3 expression, increased UNC13D expression, increased BTG1 expression, increased BTG2 expression, increased CLU expression, increased IRS1 expression, increased BCL2L expression. For example, the one or more T cells may be identified, if at least two, at least three, at least four, at least five, at least six, at least seven, or all eight of the aforementioned group of markers are increased. An increase may be at least about 2-fold, at least about 5-fold, or at least about 10-fold, preferably in comparison with a reference value. The reference value is preferably determined in endogenous T cells from the same donor (mean values). Increased expression of one or more of said markers represent the "activation signature" of the T cells. According to the invention a method to identify one or more T cells may comprise a) determining in a peripheral blood sample the mRNA expression level of one or more markers indicative for a activation signature of the T cell, which can be selected from the group consisting of GZMB, CCL3, UNC13D, BTG1, BTG2, CLU, IRS1, and BCL2L in a population of T cells; and b) identifying one or more T cells that display an activation signature by an altered expression of said one or more makers, which can be the fulfilment of at least one of the following features: increased GZMB expression, increased CCL3 expression, increased UNC13D expression, increased BTG1 expression, increased BTG2 expression, increased CLU expression, increased IRS1 expression, increased BCL2L expression. Increased expression of one or more of said markers represent the "activation signature" of the T cells. As used herein said activation signature is characteristic of activation of the T cells of the invention, namely T cells with high affinity TCRs as defined herein. The term "T cell activation" is known in the art refers to the integration of three distinct signals delivered in sequence: 1) antigen recognition, 2) costimulation (i.e. simultaneous engagement of the T-cell receptor and a co-stimulatory molecule like CD28, or ICOS, on the T cell by the major histocompatibility complex MHCII), and 3) cytokine- mediated differentiation and expansion, which leads to an effective immune response. In order to identify one or more T cells based on said activation signature, prior to determining the mRNA expression level of the markers in the peripheral blood sample, the peripheral blood sample is subjected to standard methods, defined herein, for the isolation of the PBMCs (peripheral blood mononuclear cells) present in the peripheral blood sample as defined herein. Subsequently a single-cell suspension is generated, after which the T cells are stained with fluorochrome-conjugated antibodies for flow cytometric analysis - potentially pMHC multimer staining may be included to detect antigen-specific T cells -. After this, cell sorting is performed to identify T cell receptor sequences and investigate cellular transcriptome subsequently (e.g. via single-cell RNA sequencing). Accordingly the mRNA expression levels of the gene markers defined herein may be achieved by Single-Cell RNA-Sequencing, a technique which provides transcriptional profiling of thousands of individual cells. This level of throughput analysis enables to understand at the single-cell level what genes are expressed, in what quantities, and how they differ across thousands of cells within a heterogeneous sample. In this context, the term "identifying" when referred to the one or more T cells according to the invention may therefore refer to a selection of T-cells with high affinity TCRs (defined elsewhere herein), via any method that can be used to measure (or determine) the mRNA expression level of given genes in a population of T-cells.

According to the invention, the population of T cells wherein the PD-1 levels is to be determined, or wherein the mRNA expression level of one or more of the gene markers disclosed herein is to be determined, is obtained from a peripheral blood sample derived from a subject. The term "subject" as used herein, also addressed as an individual, refers to a living mammalian organism. In some embodiment the mammal is a mouse. A subject also includes human and veterinary patients. Preferably, the term "subject" as used herein refers to a human subject. According to some embodiments, the subject from which the peripheral blood sample is obtained is a patient not yet diagnosed to suffer from cancer but is a subject suspected to be affected by cancer. According to some other embodiments, the subject is a cancer patient, meaning a human subject affected by cancer. Where the subject is a living human who may receive treatment for a disease or condition as described herein, it is also addressed as a "patient". Those in need of treatment include those already suffering from the disease.

Accordingly, a population of T cells as described herein may refer to the population of T cells found in a sample of peripheral blood. In particular, the population of T cells may be a population of PBMCs (peripheral blood mononuclear cells). The "peripheral blood sample" according to the invention may be venous peripheral blood or capillary blood. The term "venous peripheral blood" as used herein can be equivalently substituted by "whole venous blood", "whole venous peripheral blood" or "peripheral blood" and refers to the blood pool circulating throughout the body and not sequestered within the lymphatic system, spleen, liver, or bone marrow. According to the present invention, whole blood can be used for the purification (or isolation) of PMBCs. A "peripheral blood mononuclear cell" (PBMC) as described herein is any peripheral blood cell having a round nucleus. These cells consist of lymphocytes (T cells, B cells, natural killer cells) and monocytes, as opposed to erythrocytes and platelets that have no nuclei, and granulocytes which have multi-lobed nuclei. According to the present invention, said PBMCs are preferably derived from venous peripheral blood, which can be collected in 8 × 9 ml ethylene diamine tetra-acetic acid (EDTA) containing monovettes, which are part of the basic equipment found in medical practices and hospitals. Said PBMCs may be contained in capillary blood. The skilled person is aware of means and methods to prepare PBMCS from whole blood, such as venous peripheral blood or capillary blood obtained from a subject. To obtain a sufficient number of PBMC, withdrawal of several milliliters of blood is necessary. A population of peripheral blood mononuclear cells (PBMC) can be obtained by standard isolation methods such as Ficoll gradient of blood cells. Accordingly, single cell suspensions may be generated using Ficoll density gradient centrifugation to isolate cells, followed by washing steps. The cell population comprised in the sample may however also be in purified form and might have been isolated using a reversible cell staining/isolation technology as described patent in US patent 7,776,562, US patent 8,298.782, International Patent application WO02/054065 or International Patent Application WO2013/011011 which are incorporated herein by reference. Alternatively, the population of cells can also be obtained by cell sorting via negative magnetic immunoadherence as described in US Patent 6,352,694 B1 or European Patent EP 0 700 430 B1 which are incorporated herein by reference. If an isolation method described here is used in basic research, the sample might be cells of in vitro cell culture experiments. From the blood samples, the PBMCs are therefore isolated (purified) and used for determining the expression level of PD-1 in a population of T cells. Accordingly, the isolated (or purified) PBMCs are used to generate a single cell suspension. The skilled artisan is aware of methods for generating a single cell suspension. In this context, a "single cell suspension" refers to a a type of cell culture in which single cells (i.e cells that do not form aggregates between themselves) are allowed to function and multiply in an agitated growth medium, thus forming a suspension. After the single cell suspension of PBMCs is generated, the T cells are stained with fluorochrome-conjugated antibodies for flow cytometric analysis. In preferred examples, the T cells are stained with fluorochrome-conjugated antibodies specific for PD-1. A complete list of antibodies used for flow cytometric analysis of T cells is listed in Table 1. The methods used by the inventors for identifying the T cells according to the invention are described in details in the examples and in the Materials and method section of the specification. Optionally, the T cells are also stained for pMHC multimer to detect antigen-specific T cells, as disclosed elsewhere herein and described in details in the examples and materials and methods. Peptide-MHC (pMHC) multimers have become the "gold standard" for the detection and isolation of antigen-specific T-cells. The term "staining", as used herein, may refer to the use of dyes, or the use of antibodies in order to identify the presence of a specific compound in a cell or in a population of cells. In general, "staining", as it will be understood by the skilled artisan, refers to adding a class-specific (DNA, proteins, lipids, carbohydrates) dye to a substrate to qualify or quantify the presence of a specific compound.

The methods disclosed herein, further envision that after the one or more T cells are selected (either by having an expression level of PD-1 that is above the median expression of PD-1 in the population of T cells, or by having increased expression of the gene markers as disclosed herein), said T cells are subjected to further analysis such as expansion, RNA sequencing, isolation. Preferably, said T cells are PB-T cells, that is, T cells found in a peripheral blood sample, as disclosed elsewhere herein. Said PB-T cells may be therefore comprised in PBMCs as defined elsewhere herein. For example, the identified (selected) T cells may undergo two alternative approaches: in one approach the one or more identified T cells are isolated and/or expanded directly to be subsequently used for clinical adoptive T cell therapy. The skilled artisan is aware of many techniques that can be used in order to isolate T cells. T cells isolation can be done, for example, via MACS or FACS. - Magnetic-activated cell sorting (MACS) MACS^{®} (Miltenyi Biotec, Bergisch Gladbach, Germany) is a cell separation technology based on the use of monoclonal antibody-conjugated magnetic beads - . In a second approach the one or more identified T cells of interest are characterized by single-cell RNA sequencing to identify the TCR (and the transcriptomic phenotype) and then the most promising TCRs are, in a separate step, engineered into donor T cells for clinical adoptive T cell therapy. The skilled artisan is aware of different techniques for engineering TCRs into donor (i.e. host) cells. For example, the TCR may be engineered into host cells via transfection using CRISPR/Cas9 technology.

As used herein, "Adoptive cell therapy" also known as cellular immunotherapy refers to a form of treatment that uses the cells of the human immune system to eliminate cancer. Some of these approaches involve directly isolating patient's own immune cells and simply expanding their numbers, whereas others involve genetically modifying the immune cells to enhance their cancer-fighting capabilities. The adoptive cell therapy as used herein makes use of the cells of the invention.

Accordingly, the one or more T cells of the present invention are tumor-reactive T cells. By "tumor reactive T cells" as used herein it is meant T cells able to recognize and bind to tumor specific antigens, therefore, tumor reactive T cells as disclosed herein comprise but are not limited to: cytotoxic T cells and CD4 T cells. As the skilled artisan knows, a cytotoxic T cell (also known as TC, cytotoxic T lymphocyte, CTL, T-killer cell, cytolytic T cell, CD8+ T-cell or killer T cell) is a T lymphocyte (a type of white blood cell) able to eliminate cancer cells, cells that are infected (particularly with viruses), or cells that are damaged in other ways. CD8+ T-cells are therefore T cells expressing on their surface CD8. CD8 (cluster of differentiation 8) is a transmembrane glycoprotein that serves as a co-receptor for the T-cell receptor (TCR). The CD8 co-receptor is predominantly expressed on the surface of cytotoxic T cells, but can also be found on natural killer cells, cortical thymocytes, and dendritic cells. The CD8 molecule is a marker for cytotoxic T cell population. The tumor reactive T cells as disclosed herein may also encompass CD4+ T cells. CD4 (cluster of differentiation 4) is a glycoprotein that serves as a co-receptor for the T-cell receptor (TCR). CD4 is found on the surface of immune cells such as T helper cells, monocytes, macrophages, and dendritic cells. Such T cells expressing CD4 on their surface are referred to as CD4+ T cells.

The one or more T cells identified by the methods of the invention preferably comprise a high affinity TCR. An important parameter to describe T cell functionality is the affinity of their TCRs for a given antigen peptides bound to major histocompatibility complex (MHC) molecules. T cells with high affinity TCRs could be shown to be superior in clearing viral infections or inducing tumor regression, in this sense, high affinity TCR positively correlates with high tumor protection (also referred to as "high protective capacity" as used herein) as shown for example in Example 5. In this context, by tumor protection it is meant the ability of T cells of the invention to cause tumor regression. Tumor protection can be measured with many techniques known to the skilled artisan starting from preclinical mouse models wherein survival of animals is measured (as described in Example 4 and Figure 4) up to clinical studies in humans. Affinity of a T cell TCR can be assessed as the binding strength of the TCR to its cognate ligand, the peptide major histocompatibility complex (pMHC). In general, a high affinity TCR is a TCR with above-average affinity for antigen peptides bound to major histocompatibility complex (MHC) molecules. Affinity is defined as the probability of a receptor (TCR)-ligand (pMHC) interaction and is one of the most commonly used measurements to predict the T cell response to antigen. As with all receptor-ligand interactions, affinity, or the association constant (*K*ₐ) is derived from the on-rate (*k*ₒₙ) and off-rate (*k*_{off}) of receptor-ligand equilibrium and is calculated independently of concentrations of ligand and receptor (affinity = *K*ₐ = *k*ₒₙ/*k*_{off}). The affinity of an interaction is not its bond strength, or the force needed to break the bond, though these terms are often incorrectly used interchangeably.

Accordingly, the invention also relates to a method of isolating one or more T cell as defined herein, comprising isolating one or more T cells identified by the methods of the invention. Isolation of said T cell (population) can be performed by any method know in the art, for example by using a reversible cell staining/isolation technology as described patent in US patent 7,776,562, US patent 8,298,782, International Patent application WO02/054065 or International Patent Application WO2013/011011 which are incorporated herein by reference. After their isolation, the one or more T cells, may be further expanded, in order to generate a population of T cells. In this context "expansion" of one or more T cells refers to protocols wherein the T cells are allowed to proliferate, thereby forming a population of T cells. Various protocols of T cells expansion are known to the skilled artisan. Said protocols may be protocols useful for *in vitro* expansion of T cells, for example, expansion of one or more T cells isolated by the methods of the invention that might be directly expanded after their isolation. Alternatively, the T cells isolated by the method of the invention may be subjected to further analysis such as single-cell RNA sequencing (to identify the TCR sequences and the transcriptomic phenotype), after which the most promising TCRs thereby individuated are subsequently engineered into donor T cells, (or host cells, as defined herein)

In addition to the method of identifying one or more T cells disclosed herein, the inventors also developed a method of identifying one or more TCRs, comprising determining the sequence of the TCR of the one or more T cells. Accordingly, after identifying one or more T cells by the method of the invention (i.e. based on PD-1 expression levels and/or the activation signature), these T cells may be subjected to further analysis in order to determine the sequence of their TCR. Said further analysis may comprise any method known to the skilled artisan, which allows determining the nucleotide sequence encoding for any given TCR and/or determining the amino acid sequence of any given TCR. For example, the TCR sequences may be determined by single cell RNA sequencing (scRNA seq) as used in Example 6 and shown in Figure 7. scRNA seq allows to capture the TCR sequence and full RNA transcriptome of the one or more T cells identified by the methods disclosed herein. The inventors could provide methods that can identify TCR sequences and characterize their affinity at the same time, in fact, as disclosed elsewhere herein and in Examples 4 and 5, they surprisingly found that PD-1 expression levels, stratified TCRs according to their functional potential - but only outside the tumor (Fig. 5e) i.e they discovered that in peripheral blood samples high PD-1 expression correlates with high affinity TCR. Accordingly, protective capacity of TCRs strongly correlated with PD-1 MFI (Figure 5 b and 5e) for T cells outside the tumor, but not for TIL (Example 6 Fig. 5f). These data suggested that PD-1 could serve as a surrogate marker for TCRs with high affinity and high protective capacity in peripheral blood, a source that is widely accessible in patients.

Accordingly, the invention also comprises a nucleic acid molecule comprising a nucleic acid sequence encoding the TCR identified by the methods of the invention. A nucleic acid of the invention may be any nucleic acid in any possible configuration, such as single stranded, double stranded or a combination thereof. Nucleic acids include for instance DNA molecules, RNA molecules, analogues of the DNA or RNA generated using nucleotide analogues or using nucleic acid chemistry, locked nucleic acid molecules (LNA), and protein nucleic acids molecules (PNA). DNA or RNA may be of genomic or synthetic origin and may be single or double stranded. Such nucleic acid can be e.g. mRNA, cRNA, synthetic RNA, genomic DNA, cDNA synthetic DNA, a copolymer of DNA and RNA, oligonucleotides, etc. A respective nucleic acid may furthermore contain non-natural nucleotide analogues and/or be linked to an affinity tag or a label. A nucleic acid molecule comprising a nuclei acid sequence encoding the TCR according to the invention may be included in a vector such as a plasmid.

A nucleic acid molecule encoding TCR according to the invention can be expressed using any suitable expression system, for example in a suitable host cell or in a cell-free system. The obtained TCR is enriched by means of selection and/ or isolation. Thus, the nucleic acid molecule of the present invention may be comprised in a host cell or the vector comprising the nucleic acid molecule of the present invention may be comprised in a host cell.

Therefore, the invention also encompasses a method of generating one or more immune cells as described herein, comprising introducing one or more nucleic acid molecules encoding one or more TCRs, identified by the methods of the invention, into one or more host cells. In this context, the generated immune cell is preferably an immune cell suitable for adoptive T cell therapy as defined herein. The one or more nucleic acid molecules encoding one or more TCR may be introduced into one or more host cells via transduction or transfection. The term "transfection" refers to the process of introducing naked or purified nucleic acids into eukaryotic cells. Said naked or purified nucleic acids can be in the form of a vector. Accordingly, in the context of the invention, oligonucleotides encoding the heterologous T-cell receptors (TCRs) are transfected into said host cells. As disclosed herein, the TCR may be engineered into host cells via transfection using CRISPR/Cas9 technology. Alternatively, a host cell of the invention can be modified by transduction. The term "transduction" refers to the process by which foreign DNA is introduced into a cell by a virus or viral vector. Viral vectors suitable for said transfection are known to the skilled artisan.

Accordingly, suitable host cells for the expression of the isolated TCRs may be preferably eukaryotic, preferably human host cells which are suitable to be transferred into a subject as defined herein for adoptive T cell therapy. Suitable host cells may by immune effector cells, preferably human immune effector cells, i.e. cells from the human body that have differentiated into a form capable of modulating or effecting an immune response. The host cells of the invention may be heterologous or autologous cells. In this context the term "heterologous" when applied to the host cell of the invention refers to a cell which is not derived from the subject as defined herein, while the term "autologous" when applied to the host cell of the invention refers to a cell which is derived from the subject as defined herein. Additionally, the host cell of the invention may be immortalized. An "immortalized" cell, or cell line, is a cell or a population of cells from a multicellular organism which would normally not proliferate indefinitely but, due to mutation, have evaded normal cellular senescence and instead can keep undergoing division. The cells can therefore be grown for prolonged periods *in vitro.* The mutations required for immortality can occur naturally or be intentionally induced for experimental purposes. Examples of immortalized cells include HeLa cells - a widely used human cell line isolated from cervical cancer patient Henrietta Lacks; HEK 293 cells - derived from human fetal cells; Jurkat cells - a human T lymphocyte cell line isolated from a case of leukemia; OK cells - derived from female North American opossum kidney cells; Ptk2 cells - derived from male long-nosed potoroo epithelial kidney cells; Vero cells - a monkey kidney cell line that arose by spontaneous immortalization. Preferred immortalized cells are Jurkat cells. The host cell of the invention may also be an iPSC (induced Pluripotent Stem Cell). iPSCs are known to the skilled artisan, and they are a type of pluripotent stem cell that can be generated directly from a somatic cell. Preferably the host cell of the invention is an immune cell, preferably a human immune cell. Additionally, the host cell of the invention may be a T cell. In preferred embodiments the cell of the human immune system is a CD8+ T cell or a CD4+ T cell. The host cell according to the invention may also be a gamma-delta T cell, or a NK-T cell. Gamma delta T cells (γδ T cells) are T cells that have a distinctive T-cell receptor (TCR) on their surface. Most T cells are αβ (alpha beta) T cells with TCR composed of two glycoprotein chains called α (alpha) and β (beta) TCR chains. In contrast, gamma delta (γδ) T cells have a TCR that is made up of one γ (gamma) chain and one δ (delta) chain. This group of T cells is usually less common than αβ T cells. Natural killer T (NKT) cells are a heterogeneous group of T cells that share properties of both T cells and natural killer cells. Many of these cells recognize the non-polymorphic CD1d molecule, an antigen-presenting molecule that binds self and foreign lipids and glycolipids. They constitute only approximately 1% of all peripheral blood T cells. Natural killer T cells should neither be confused with natural killer cells nor killer T cells (cytotoxic T cells).

According to the invention, after introducing one or more nucleic acid molecules encoding one or more TCRs of the invention into the one or more immune cells, said cells are subjected to expansion. As defined elsewhere herein "expansion" of one or more T cells refers to protocols wherein the T cells are allowed to proliferate, thereby forming a population of T cells. In particular, in the context of the invention more than one TCR identified by the methods of the invention may be introduced into more than one T cell. In the context of the invention, when said more than one T cells are then subjected to expansion, they can generate a polyclonal population of T cells. A polyclonal population of T cells is obtained when said population of T cells is derived from expansion of more than one T cell (clone), wherein each clone presents a different TCR of the invention on its surface.

The present invention also relates to a pharmaceutical composition comprising the T cell identified by the method of the invention, the T cell isolated by the method of the invention, the population of T cells obtained by the method of the invention, the host cell of the invention, or one or more immune cells generated by the method of the invention. Accordingly, the term "pharmaceutical composition" relates to a composition for administration to a patient, preferably a human patient. Pharmaceutical compositions or formulations are usually in such a form as to allow the biological activity of the active ingredient (the T cells of the present invention) to be effective and may therefore be administered to a subject for therapeutic use as described herein. The pharmaceutical composition may be a composition for intraperitoneal, intravenous, intraarterial, subcutaneous, intramuscular, parenteral, transdermal, intraluminal, intrathecal and/or intranasal administration or for direct injection into tissue. Said composition may be administered to a patient via infusion or injection, preferably by injection. Administration of the suitable compositions is preferably intravenously, intraperitoneally, intraarterially, subcutaneously, intramuscularly. The pharmaceutical compositions can be administered to the subject at a suitable dose. The dosage regimen will be determined by the attending physician and by clinical factors. As is well known in the medical arts, dosages for any one patient depend upon many factors, including the patient's size, body surface area, age, the particular compound to be administered, sex, time and route of administration, general health, and other drugs being administered concurrently. In the context of the present invention the pharmaceutical composition may comprise a therapeutically effective amount of the T cell isolated by the method of the invention, the population of T cells obtained by the method of the invention, the host cell of the invention, or one or more immune cells generated by the method of the invention. Accordingly, a therapeutic effective amount of T cells according to the invention may be less than 400 T cells.

The present invention therefore envisages the T cell identified by the methods described herein, the T cell isolated by the methods described herein, the population of T cells obtained by the methods described herein, the host cell described herein, or the one or more immune cells generated by the method described herein for use in therapy. Preferably, said therapy is adoptive T cell therapy, as defined elsewhere herein. Preferably, said therapy is adoptive T cell therapy is cancer therapy. Adoptive cell therapy can entail use of autologous or heterologous immune cells. For example, in the case of an autologous cell, T cells may be isolated according to the methods of the invention from a subject. The obtained tumor-reactive T cells are expanded and subsequently transferred back to the subject. In the case of heterologous T cells, one or more immune cells generated by the method of the invention may be used, for example a host cells as defined herein may be subjected to transfer of a TCR according to the invention, and transferred to a subject for adoptive T cell therapy.

As used herein, the term "treat", "treating" or "treatment" means to reduce (slow down (lessen)), stabilize or inhibit or at least partially alleviate or abrogate the progression of the symptoms associated with the respective disease. Thus, it includes the administration of said T cell isolated by the method of the invention, the population of T cells obtained by the method of the invention, the host cell of the invention, or the one or more immune cells of the invention, preferably in the form of a medicament, to a subject, defined elsewhere herein. Those in need of treatment include those already suffering from the disease, here cancer. Preferably, a treatment reduces (slows down (lessens)), stabilizes, or inhibits or at least partially alleviates or abrogates progression of a symptom that is associated with the presence and/or progression of a disease or pathological condition. "Treat", "treating", or "treatment" refers thus to a therapeutic treatment. In particular, in the context of the present invention, treating or treatment refers to an improvement of the symptom that is associated with cancer, as defined elsewhere herein.

The present invention also relates to a method of diagnosis of cancer. The method of diagnosing accordingly may comprise measuring the PD-1 expression level in a blood sample as defined herein, and determining whether the PD-1 expression level in a population of T cells in the sample is above the expression of PD-1 in the population of T cells found in a reference blood sample. Said levels of PD-1 protein expression found in this particular type of sample would therefore indicate that the patient cells comprise T cells having high-affinity TCRs as defined herein. This finding would indicate that said patient could benefit more from checkpoint blockade with PD-1 inhibitor antibodies compared to a reference blood sample. Said reference may be (a blood sample from) a healthy subject or another patient who doesn't have such high PD-1 expressing T cells in peripheral blood samples. The determination of whether the PD-1 expression level in a test sample is above the PD-1 expression in a reference sample can further be assisted, for example, by comparing different patients and including positive controls, i.e. samples after T cell stimulation. Accordingly, the method of diagnosing cancer may be a method of selecting a patient for treatment with an PD-1 inhibitor, such as a PD-1 inhibiting antibody. The disclosure thus also envisions an PD-1 inhibitor, such as a PD-1 inhibiting antibody for use in the treatment of a patient selected by the methods disclosed herein.

The invention is further characterized by the following items.
Item 1. A method of identifying one or more T cells comprising: a. determining in a peripheral blood sample derived from a subject the expression level of PD-1 in a population of T cells; and b. identifying one or more T cells that have an expression level of PD-1 that is above the median expression of PD-1 in the population of T cells.
Item 2. The method of item 1 wherein PD-1 expression is PD-1 protein expression.
Item 3. The method of item 1 or 2, wherein the one or more T cells are identified if the one or more T cells have an expression level of PD-1 that is within about the top 20%, preferably within about the top 10%, within about the top 5%, preferably within about the top 4% of the PD-1 expression level found in said population of T cells.
Item 4. A method of identifying one or more T cells comprising: a. determining in a peripheral blood sample the mRNA expression level of one or more markers selected from the group consisting of GZMB, CCL3, UNC13D, BTG1, BTG2, CLU, IRS1, and BCL2L in a population of T cells; and b. identifying one or more T cells that fulfill at least one of the following features: i. increased GZMB expression; ii. increased CCL3 expression; iii. increased UNC13D expression; iv. increased BTG1 expression; v. increased BTG2 expression; vi. increased CLU expression; vii. increased IRS1 expression; viii. increased BCL2L expression.
Item 5. The method of item 4, wherein prior to determining the mRNA expression level in the peripheral blood sample, said peripheral blood sample is subjected to generation of single cell suspension, staining, and/or cell sorting.
Item 6. The method of any one of the preceding items, wherein the method is for identifying one or more tumor-antigen specific T cells.
Item 7. The method of any one of the preceding items, wherein the peripheral blood sample is derived from a human subject.
Item 8. The method of any one of the preceding items, wherein the subject has or is suspected to have cancer.
Item 9. The method of any one of the preceding items, wherein the peripheral blood sample is a peripheral blood mononuclear cell (PBMC) sample.
Item 10. The method of any one of the preceding items, further comprising selecting the one or more T cells identified in step b. for further analysis.
Item 11. The method of any one of the preceding items, further comprising isolating the one or more T cells identified in step b.
Item 12. The method of any one of the preceding items, further comprising sequencing the one or more T cells identified in step b.
Item 13. The method of any one of the preceding items, further comprising expanding the one or more T cells identified in step b.
Item 14. The method of any one of the preceding items, wherein the one or more T cells are peripheral blood T cells (PB-T cells).
Item 15. The method of any one of the preceding items, wherein the one or more T cells are tumor-reactive T cells.
Item 16. The method of any one of the preceding items, wherein the one or more T cells are cytotoxic T cells.
Item 17. The method of any one of the preceding items, wherein the one or more T cells comprise a high affinity TCR that is preferably specific for a tumor target.
Item 18. A method of isolating one or more T cell, comprising isolating one or more T cells identified according to a method of any one of items 1-17.
Item 19. The method of item 18, further comprising expanding the one or more T cells.
Item 20. A method of identifying one or more T cell receptors (TCR) comprising determining the sequence of the TCR of the one or more T cells identified by the method of any one of items 1-17.
Item 21. A nucleic acid molecule comprising a nucleic acid sequence encoding the TCR identified by the method of item 20, or encoding a TCR comprised in a T cell identified by the method of any one of items 1-17.
Item 22. A method of generating one or more immune cells comprising introducing one or more nucleic acid molecules encoding one or more TCRs or antigen-binding fragments thereof, identified by the method of item 20 into one or more host cells.
Item 23. The method of item 22, wherein the host cell is an immune cell.
Item 24. The method of item 22 or 23, wherein the host cell is an immune effector cell.
Item 25. The method of any one of items 22-24, wherein the host cell is a T cell.
Item 26. The method of any one of items 22-25, wherein the host cell is a cytotoxic T cell.
Item 27. The method of any one of items 22-25, wherein the host cell is a gamma-delta T cell.
Item 28. The method of any one of items 22-24, wherein the host cell is an NK-T cell.
Item 29. The method of item 22, wherein the host cell is a stem cell, preferably an induced pluripotent stem cell (iPSC).
Item 30. The method of any one of items 22-29, wherein the host cell is a heterologous cell.
Item 31. The method of any one of items 22-29, wherein the host cell is autologous cell.
Item 32. The method of any one of items 22-31 wherein the host cell is immortalized.
Item 33. The method of item 20-32 wherein the host cell is a human cell.
Item 34. The method of any one of items 22-33, further comprising expanding the one or more immune cells.
Item 35. The method of any one of items 22-23, wherein the method comprises introducing more than one TCR identified by the method of item 20 into more than one host cell.
Item 36. A host cell comprising a nucleic acid molecule of item 21 or generated by the method of any one of items 22-35.
Item 37. A pharmaceutical composition comprising a T cell identified by the method of any one of items 1-17 or isolated by the method of item 18 or 19, or an immune cell generated by the method of any one of items 22-35, or a host cell of item 36.
Item 38. A T cell identified by the method of any one of items 1-17 or isolated by the method of item 18 or 19, or an immune cell generated by the method of any one of items 22-35, or a host cell of item 36, for use in therapy.
Item 39. The T cell, immune cell, or host cell for the use of item 38, wherein the therapy is adoptive T cell therapy.
Item 40. The T cell, immune cell, or host cell for the use of item 38 or 39, wherein the use is in the treatment of cancer.
Item 41. A method of diagnosing cancer in a subject comprising measuring the PD-1 expression level in a peripheral blood sample obtained from the subject, wherein an elevated PD-1 expression level as compared to a reference peripheral blood sample from a healthy control indicates the presence of T cells having high affinity TCRs.

Unless otherwise stated, the following terms used in this document, including the description and claims, have the definitions given below.

Those skilled in the art will recognize, or be able to ascertain, using not more than routine experimentation, many equivalents to the specific embodiments of the invention described herein. Such equivalents are intended to be encompassed by the present invention.

It is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent applications, scientific publications, manufacturer's specifications, instructions, etc.) are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### EXAMPLES

**Example 1: Tracking TCR-transgenic T cells.** The inventors first set out to examine if they can track low numbers of transferred T cells over space and time, and which phenotypic adaptation tumor-specific T cells undergo during the course of the anti-tumor immune response. To this end, the inventors performed total body irradiation (TBI) on C57BL/6 mice and transferred 5.000 naïve (CD44lo) CD8+ CD45.1+ OT-I T cells i.p., the next day followed by s.c. inoculation of MC38 OVA tumor cells, thereby making use of the well characterized syngeneic MC38 OVA tumor model40-43. Despite being a transplantable tumor model, recent evidence demonstrates MC38 OVA-induced reversible changes on systemic immunity, and a tumor microenvironment that resembles human tumors particularly well (44). The OT-I TCR recognizes the target H2kb/SIINFEKL - which is a neoepitope45 in this setting - with high affinity (46). On day 7, 12 and 17 after transfer, OT-I T cells were readily detectable by flow cytometry in peripheral blood, draining (dLN) and non-draining (ndLN) lymph nodes, as well as among TILs (Fig. 1a). Transferred T cells represented about 70% of CD8+ TILs and about 30% of all peripheral CD8+ T cells in peripheral blood at the peak of the response on day 12 (Fig. 1b). Quantification of absolute cell numbers revealed an early abundance of transferred T cells in dLN compared to ndLN at day 7, whereas OT-I cells spread out systemically into ndLN only at later time points. The inventors next wondered whether an adoptive transfer of ultra-low cell numbers could also lead to readily detectable progeny in this setting. Remarkably, the adoptive transfer of 6.5-fold less cells (i.e. 800 OT-I cells) did not change the size of progeny at day 12, and even a further reduction of the cell dose by 6.5-fold (i.e. 128 OT-I cells) only led to minor reduction of OT-I cells found among TILs (Fig. 1c). These results hinted at the possibility of investigating the spatiotemporal fate of tumor-specific T cells arising from physiologically low precursor numbers.

**Example 2: Site-dependent PD-1 expression levels indicate differential phenotypes of tumor specific T cells.** T cell exhaustion markers are relevant in two ways. One the one hand, they indicate the cellular differentiation state of tumor-specific T cells, thereby contributing to a basic understanding on the evolution of tumor-specific T cell immunity (23 Bos, R., Marquardt, K. L., Cheung, J. & Sherman, L. A. Functional differences between low- and high-affinity CD8 + T cells in the tumor environment. Oncoimmunology 1, 1239-1247 (2014), 26. Martínez-Usatorre, A., Donda, A., Zehn, D. & Romero, P. PD-1 Blockade Unleashes Effector Potential of Both High- and Low-Affinity Tumor-Infiltrating T Cells. J. Immunol. ji1701644 (2018) doi:10.4049/jimmunol.1701644. 27. Caserta, S., Kleczkowska, J., Mondino, A. & Zamoyska, R. Reduced Functional Avidity Promotes Central and Effector Memory CD4 T Cell Responses to Tumor- Associated Antigens. J. Immunol. 185, 6545-6554 (2010)). On the other hand, exhaustion markers such as PD-1 represent potential surrogate markers for guiding the use of checkpoint inhibitor therapy12 and the identification of tumor antigen-specific T cells (13. Gros, A. et al. PD-1 identifies the patient-specific CD8+ tumor-reactive repertoire infiltrating human tumors. J. Clin. Invest. 124, 2246-59 (2014). 14. Gros, A. et al. Prospective identification of neoantigen-specific lymphocytes in the peripheral blood of melanoma patients. Nat. Med. (2016) doi:10.1038/nm.4051). The inventors checked adoptively transferred OT-I cells as well as endogenous CD8+ T cells for the expression of PD-1, TIM-3 and LAG-3 (Fig. 1d-f). Transferred T cells were mostly PD-1+, TIM-3+ and/or LAG-3+ across all sites (Fig. 1e). Interestingly, at day 7, the majority of endogenous CD8+ T cells were PD-1+ or TIM-3+. While this population contracted or downregulated PD-1 and TIM-3 from then onwards, OT-I TILs remained highly positive especially for PD-1, but also for TIM-3 and LAG-3. Of note, the transferred cell number did not influence the expression pattern of any of the investigated co-inhibitory markers. Several studies have established that intermediate levels of PD-1 indicate recently activated T cells, whereas high levels of PD-1 indicate exhausted T cells 15. Thommen, D. D. et al. A transcriptionally and functionally distinct PD-1 + CD8 + T cell pool with predictive potential in non-small cell lung cancer treated with PD-1 blockade. Nat. Med. (2018) doi:10.1038/s41591-018-0057-z. In the inventor's model, the PD-1 MFI was higher in OT-I TILs compared to OT-I cells in peripheral blood (Fig. 1f). In fact, PD-1 expression levels followed a consistent site-dependent gradient, being highest in TILs, second highest in dLN, third-highest in ndLN and lowest in peripheral blood. A further hallmark of T cell exhaustion is the simultaneous expression of multiple co-inhibitory receptors48. PD-1/TIM-3/LAG-3 triple positivity of OT-I T cells (Fig. 1g) followed the same site-dependent pattern as PD-1 expression levels did (Fig. 1f). Accordingly, PD-1 MFI showed a strong linear correlation with co-expression of co-inhibitory receptors (Fig. 1h). Overall, PD-1 expression levels correlated with global upregulation of co-inhibitory receptors and at the same time markedly differed depending on the T cell site, indicating signatures of T cell exhaustion in the tumor and of recent T cell activation in peripheral blood.

**Example 3: Ultra-low T cell numbers can mediate tumor protection.** The inventors next tested whether the T cells for which they detected the described spatio-temporal phenotypic adaptations were of functional importance, or whether in immunocompetent C57BL/6 mice the addition of low numbers of high-affinity T cells to the endogenous response was irrelevant for tumor protection. To this end, they sorted with high purity and adoptively transferred 128, 320, 800, 2.000 or 5.000 naïve (CD44lo) CD8+ CD45.1+ OT-I T cells, or - as negative controls - 5.000 P14 T cells, which recognize the LCMV antigen gp33. 2.000 or 5.000 OT-I T cells conveyed almost perfect protection, whereas the endogenous immune response was unable to mediate any protection when only antigen-unspecific P14 cells were transferred in addition (Fig. 2). Remarkably, as few as 128 OT-I T cells showed a partially protective effect, the latter becoming increasingly apparent with higher cell doses (Fig.2). Thus, in the model of the inventors a cell dose that almost perfectly matches physiological precursor numbers for H2kb/SIINFEKL in naïve T cell repertoires (Thommen, D. D. et al. A transcriptionally and functionally distinct PD-1 + CD8 + T cell pool with predictive potential in non-small cell lung cancer treated with PD-1 blockade. Nat. Med. (2018) doi:10.1038/s41591-018-0057-z.), could mediate partial tumor protection.

**Example 4: A SIINFEKL-specific TCR library provides TCRs with distinct profiles of *in vitro* and *in vivo* functionality.** With this system at hand, the inventors aimed to investigate the impact of TCR affinity on tumor protection and spatiotemporal T cell fate. In the past, OT-I T cells have frequently been used for such purposes, often together with altered peptide ligands (APLs) of SIINFEKL to cover differential affinity TCR-epitope ligand interactions_{26,49}. However, despite its wide usage, the OT-I TCR has some unconventional features_{50,51}, which argues for the importance of studying different TCRs (best with a gradient of the parameter under investigation, e.g. TCR-pMHC affinity) before drawing general conclusions. Furthermore, the APL system is prone to biases through differences in peptide-pMHC affinity and/or stability₅₂. Finally, most T cell studies use adoptive transfers of monoclonal populations. While this reflects the clinical situation of adoptive cell therapy, the translation of findings to physiological T cell responses, which are polyclonal, is questionable in this setting. For these reasons the inventors previously identified a repertoire of 15 unique H2k_{b}/SIINFEKL-specific TCRs with differential structural affinity, and developed a polyclonal transfer system that allows *in vivo* monitoring of defined composite TCR repertoires during the course of an immune response₃₇. To study TCR-dependent tumor-specific immunity, the inventors chose four TCRs - TCR16-30, TCR9, TCR28 and TCR39 - which represented the entire affinity spectrum and first performed further in-depth TCR characterization experiments (Fig. 3; note that several experiments were also validated by additional TCRs). TCRs were genetically re-expressed in congenically marked hematopoetic stem cells (HSCs) and these HSCs were transferred into irradiated mice, to give rise to CD45.1+ physiological naïve T cell populations expressing a given TCR (so-called TCR-retrogenic mice) (Schober, K. et al. Reverse TCR repertoire evolution toward dominant low-affinity clones during chronic CMV infection. Nat. Immunol. 21, 434-441 (2020)). While CD8 (Fig. 3a-b), CD27, CD62L and CD44 expression did not differ between the TCRs, differential pMHC MFI pointed towards a potential gradient in TCR affinity (Fig. 3a-b). However, it is controversial how well pMHC MFI correlates with TCR affinity₃₀. Instead, *in vivo* expansion to antigenic stimulus and peptide sensitivity represent more reliable measures of TCR functionalityss. The inventors therefore first tested how 100 CD45.1₊ naïve TCR-retrogenic T cells expand in response to infection with *Listeria monocytogenes* (*L.m.*) OVA. TCR16-30 T cells expanded most (expansion was comparable to OT-I T cells (Buchholz, V. R. et al. Disparate individual fates compose robust CD8+ T cell immunity. Science 340, 630-5 (2013)), TCR9 and TCR28 T cells expanded less (for both TCRs, T cells expanded to a similar degree) and TCR39 T cells expanded least (Fig. 3c). All TCR-retrogenic T cells released IFNγ, TNFα and IL-2 upon antigen-specific stimulation *ex vivo* (Fig. 3d-e), including a considerable share of polyfunctional T cells co-releasing two or all three of the investigated cytokines. 40% of TCR16-30 T cells robustly released IFNγ already at very low peptide doses (Fig. 3e). In line with this, TCR 16-30 showed the highest peptide sensitivity of all 15 TCRs, even slightly surpassing the OT-I receptor (Fig. 3f-g). The inventors also tested the peptide sensitivity of OT-I towards the SIINFEKL APL SIITFEKL (T4) in the same system for contextualization purposes (Fig. 3g). The OT-I/T4 interaction marks the threshold for thymic selection (Enouz, S. et al. Autoreactive T cells bypass negative selection and respond to selfantigen stimulation during infection. J Exp Med 209, 1769-1779 (2012)) and is one of the lowest TCR affinities for which peptide sensitivity can be reliably analyzed. Compared to the extremely low peptide sensitivity of the OT-I towards T4, it becomes apparent that TCR39 has a markedly lower peptide sensitivity than most of the other TCRs, but is not of ultra-low functionality. Along these lines, more than 10% of TCR39 T cells started to release cytokines in response to very low amounts of antigen (Fig. 3e). Across the TCRs, mean peptide sensitivity correlated strongly with *in vivo* expansion in response to *L.m.* OVA (Fig. 3h). The inventors next tested the ability of T cells equipped with a defined TCR to kill tumor cells *in vitro* (Fig. 3i-j). TCR16-30 T cells showed a moderately, but consistently better killing capacity than TCR39 T cells did (Fig. 3i-j). Killing capacity could be titrated with cell dosages and - as for peptide sensitivity (Fig. 3g) - TCR16-30 performed slightly better than OT-I T cells, underlining that TCR16-30 has a very high functional potential. *In vivo*, TCR16-30 and TCR39 T cells both mediated tumor protection in a dose-dependent manner (Fig. 4a). Thereby, only TCR16-30 T cells mediated perfect protection, for which as few as 800 T cells sufficed. While 80% survival of animals could be reached with 5.000 TCR39 T cells, only 320 TCR16- 30 T cells were required for the same effect. Since 320 and 800 T cells seemed to differentiate the protective capacity of TCRs in the most sensitive manner, the inventors performed another experiment with the same cell doses to compare TCR16-30, TCR9, TCR28 and TCR39 side-by-side (Fig. 4b). The investigated TCRs yielded protection exactly as predicted by pMHC staining, *in vivo* response to *L.m.* OVA infection, peptide sensitivity and *in vitro* tumor cell killing capacity (Fig. 3). Again, ultra-low cell numbers of TCR16-30 sufficed for perfect protection.

**Example 5: In polyclonal populations, PD-1 is a surrogate marker for TCRs with high protective capacity outside the tumor.** Having established that TCR16-30 is a highly protective TCR, the inventors wondered whether T cells with this TCR would show distinct features in terms of T cell homing, abundance or phenotypic adaptation within polyclonal populations harboring also TCRs with gradually worse functionality, as it is the case in physiological T cell responses. To this end, the inventors generated retrogenic mice for each TCR (Fig. 5a-b) with a unique congenic marker background of CD45.1/.2 and CD90.1/.2 alleles (Schober, K. et al. Reverse TCR repertoire evolution toward dominant low-affinity clones during chronic CMV infection. Nat. Immunol. 21, 434-441 (2020), 5454. Grassmann, S. et al. Distinct Surface Expression of Activating Receptor Ly49H Drives Differential Expansion of NK Cell Clones upon Murine Cytomegalovirus Infection. Immunity 1-10 (2019) doi:10.1016/j.immuni.2019.04.015.) and transferred and monitored the progeny of 500 naïve CD8+ T cells for each TCR within the same mice after tumor inoculation (Fig. 5c).With the help of isotype-specific antibodies, the inventors were able to identify T cell populations for each TCR in TIL, dLN, ndLN and peripheral blood. Surprisingly, the functionality of TCRs did not seem to have any consistent consequence on homing or abundance of TCR-retrogenic T cells in any of the investigated tissues (Fig. 5d). Since PD-1 has been postulated as a surrogate marker of tumor antigen-specific T cells_{13,14}, the inventors next wondered whether PD-1 expression would be different on T cells with TCRs of distinct protective capacity. As initially observed for OT-I (Fig. 1), for all TCRs tumor antigen-specific T cells were nearly completely PD-1+ in the tumor and mostly PD-1+ outside the tumor (Fig. 5e). Also, as seen before, tumor antigen-specific T cells showed a high PD-1 MFI in the tumor, but differential and more intermediate PD-1 expression levels in dLN, ndLN and peripheral blood. Intriguingly, PD-1 positivity, and especially PD-1 expression levels, stratified TCRs according to their functional potential - but only outside the tumor (Fig. 5e). Accordingly, protective capacity of TCRs upon monoclonal transfer (data from Fig. 5b) strongly correlated with PD-1 MFI upon polyclonal transfer (data from Fig. 5e) for T cells outside the tumor, but not for TIL (Fig. 5f). These data suggested that PD-1 could serve as a surrogate marker for TCRs with high protective capacity in peripheral blood, a source that is usually widely accessible in patients. To explore this in further detail, the inventors gated on different fractions of PD-1 expressing CD8+ T cells, including both transferred and endogenous T cells (Fig. 6a). The inventors then performed an analysis of the coverage of each individual population for cumulative PD-1 FI ranks with single-cell resolution (Fig. 6b, top left panel for each tissue). For this, the inventors defined the top PD-1 expressing fraction for each mouse individually (as in top 20% PD-1 expressing fraction in mouse 1, top 20% PD-1 expressing fraction in mouse 2, etc) and not based on defined PD-1 MFI thresholds. For all observations, however, similar results were obtained using defined PD- 1 MFI thresholds (data not shown). In the tumor, the top 20% PD-1 expressing cells contained almost all cells harboring TCR16-30, TCR9 and TCR28 (Fig. 6b, top left panel for TIL). TCR39, the least protective TCR, was mostly contained in the top 40% PD-1 expressing cells. In peripheral blood, this pattern was different. While TCR-retrogenic T cells were also enriched among the top PD-1 expressing cells in comparison to the endogenous population, saturation of coverage was only reached when almost all cells were gated on (Fig. 6b, top left panel for peripheral blood). Thus, within the dichotomous distribution of PD-1 expression in the tumor (Fig. 6a), antigen-specific T cells are almost exclusively found in the PD-1+ fraction, which is PD-1ₕᵢ (also see Fig. 5e). In contrast, outside the tumor, antigen-specific T cells were more widely distributed across a spectrum of PD-1 expression. Still, in comparison to endogenous CD8₊T cells, transferred T cells were generally enriched among cells with top PD-1 expression levels in all tissues. This confirms that PD-1 expression can guide the identification of tumor antigen-specific T cells within the tumor and in peripheral blood (Gros, A. et al. PD-1 identifies the patient-specific CD8+ tumor-reactive repertoire infiltrating human tumors. J. Clin. Invest. 124, 2246-59 (2014); Gros, A. et al. Prospective identification of neoantigen-specific lymphocytes in the peripheral blood of melanoma patients. Nat. Med. (2016) doi:10.1038/nm.4051). the inventors then further analyzed the top PD-1 expressing cells in peripheral blood (whose intermediate PD-1 expression levels are just enough to make them partly PD-1+, Fig. 6a, Fig. 5e). the inventors plotted the abundance of each subpopulation among all CD8+ T cells for cumulative PD-1 FI ranks (Fig. 6b, bottom left panel for each tissue). Strikingly, in peripheral blood, the highly protective TCR16-30 was significantly enriched among the top PD-1 expressing T cells. In contrast, TCR16-30 was not enriched in any PD-1 fraction in the tumor. In-depth analysis of the coverage and abundance of each transferred population for cumulative PD-1 FI ranks revealed that it is particularly within the top 4% PD-1 fraction that TCR16-30 T cells are enriched outside the tumor. Specifically, in the top 4% PD-1 fraction of transferred cells, 80% of cells expressed TCR16-30, whereas TCR16-30 T cells were not more abundant than any of the other TCR-retrogenic T cells when PD-1 was not used for pre-gating in peripheral blood and were not enriched in any PD-1 FI rank among TIL (Fig. 6b, bottom right panel for each tissue). Overall, these data point towards PD-1 as a surrogate marker for TCRs with high protective capacity outside the tumor.

**Example 6: High-functionality neoantigen-specific TCRs can be identified in peripheral blood of a human melanoma patient based on phenotypic traces of recent activation.** The inventors next aimed to investigate whether their findings would hold true for human tumor-specific T cells *in vivo.* To this end, the inventors analyzed T cells specific for the HLA-A*01:01-restricted neoantigen ENTPD4_{P>L}, in which the wildtype sequence ATDTNNPNVNY is mutated to ATDTNNLNVNY, in a human melanoma patient₅₇. This patient was a 44-year old woman with a stage M1b BRAF V600E mutation positive melanoma with lymphadenopathy and subcutaneous and soft tissue metastases. Her previous treatment included the CTLA-4 targeting checkpoint inhibitor ipilimumab. The patient received 7×10₁₀ TIL with two doses of IL- 2, which induced partial remission. Partial remission lasted for 6 months, and the patient survived in total for 16 months after TIL treatment. T cells reactive to ENTPD4_{P>L} constituted 5.06% of CD8 T cells in the TIL infusion product and could also be identified in peripheral blood before (from here on referred to as 'PB-pre') and 212 days after (from here on referred to as 'PB-post') TIL treatment (constituting 0.74% and 0.26% of CD8 T cells, respectively; Fig. 6a) (van den Berg, J. H. et al. Tumor infiltrating lymphocytes (TIL) therapy in metastatic melanoma: boosting of neoantigen-specific T cell reactivity and long-term follow-up. J. Immunother. Cancer 8, e000848 (2020)). To investigate TCR-dependent phenotypic adaptation inside and outside the tumor, the inventors performed single-cell RNA sequencing (scRNA seq), capturing the TCR sequence and full RNA transcriptome, of ENTPD4_{P>L}-specific, pMHC multimer₊ CD8 T cells sorted by flow cytometry. Uniform manifold approximation projection (UMAP) (Becht, E. et al. Dimensionality reduction for visualizing single-cell data using UMAP. Nat. Biotechnol. 37, 38-44 (2019)) and Leiden clustering (Traag, V. A., Waltman, L. & van Eck, N. J. From Louvain to Leiden: guaranteeing well connected communities. Sci. Rep. 9, 5233 (2019)) of sorted T cells based on their full transcriptome showed sample-dependent clustering, with PBpost T cells clustering in-between PB-pre T cells and the TIL infusion product itself (Fig. 6b), consistent with shaping of PB-post T cell phenotypes through infused TIL. To first analyze the phenotypic profile of ENTPD4_{P>L}-specific T cells in an unbiased manner, the inventors investigated the top 5 differentially expressed genes of a given Leiden cluster compared to all other clusters. Leiden cluster 3, which was largely formed by PB-pre T cells (Fig. 6c), was characterized by expression of genes associated with cytotoxic T cells, such as *GZMH*, *NKG7* and CCL5. In addition, the fibroblast growth factor-binding protein 2 *(FGFBP2)* was exclusively expressed by Leiden cluster 3 (PB-pre) T cells. *FGFBP2* has previously been described to be a core gene of tumor-specific T cells with a 'cytotoxic signature' in several independent studies₆₀. Leiden cluster 4, which was largely constituted by PB-post T cells, was characterized by expression of CD127-encoding *IL7R* and the Thioredoxin Interacting Protein encoding *TXNIP.* While *IL7R* is clearly associated with naïve-like T cells, *TXNIP* has been described for naïve-like, cytotoxic and predysfunctional cells. the inventors were intrigued by the fact that this analysis revealed key genes from previously defined signatures of tumor-specific T cells ('cytotoxic', 'predysfunctional', 'dysfunctional' and 'naïve-like' (van der Leun, A. M., Thommen, D. S. & Schumacher, T. N. CD8+ T cell states in human cancer: insights from single-cell analysis. Nat. Rev. Cancer 20, 218-232 (2020)), and next checked for the expression of signature-defining genes in all three sample types. This showed that PB-pre T cells showed a 'cytotoxic' phenotype, while TIL showed a 'dysfunctional' phenotype (Fig. 6d-e). Of note, while most co-inhibitory molecules that defined the 'dysfunctional' signature such as *LAG3*, *LAYN*, *HAVCR2* and *CTLA4* were markedly enriched among TIL, *PDCD1* transcript was higher in PB-pre T cells compared to TIL (Fig. 6e). This at first sight seemed to contradict findings of higher PD-1 protein expression in mouse T cells in the tumor compared to in peripheral blood. However, recent data also described PD-1 protein, but not *PDCD1* RNA levels, to be predictive of tumor antigen specificity among peripheral blood T cells, making it likely that PD-1 protein and *PDCD1* RNA levels are disconnected. The cytotoxic signature of tumor-specific T cells in peripheral blood suggested that these T cells could show signs of recent activation, similar to our findings in the pre-clinical model. The inventors therefore also analyzed T cells from all tissue sites for the expression of a general T cell 'activation score' (ref). Similar to the cytotoxic signature, PB-pre T cells scored highest for this activation phenotype (Fig. 6d-e). Overall, our analyses confirmed that T cells specific for the same tumor epitope have markedly different phenotypes dependent on the sampling site, with 'dysfunction' being a phenotype most prominent among T cells in the tumor, whereas tumor specific T cells outside the tumor rather show 'cytotoxic' or 'naïve-like' phenotypes and an 'activation' signature. The inventors next aimed to investigate whether the abundance and phenotypes were consistent or unique across different clonotypes per sample site. The inventors identified paired αβ TCR sequences in 78.9 % of T cells and subsequently concentrated our analyses on these clonotypes (see methods for clonotype definition). This corresponded to 28 unique TCRs that the inventors found in at least 2 cells, of which 2 TCRs were exclusively found in PB-re, 15 TCRs exclusively in TIL and no TCR exclusively in PB-post T cells. 4 TCRs were shared between PB-pre and TIL, 9 TCRs were shared between TIL and PB-post and only 1 TCR was shared between PB-pre and PB-post. 7 TCRs were found in all three sampling sites. This indicated both overlap and unique TCR niches for the sampling sites, with more overlap between TIL and PB-post compared to PB-pre and TIL. Accordingly, while clonotype sizes between TIL and PB-post T cells correlated well, PB-pre T cells had a unique size distribution of clonotypes. In particular, TCR4 was the most dominant TCR among PB-pre T cells, but almost absent among TIL and completely absent among PB-post T cells (Fig. 6f). Vice versa, TCRs 14 and 2, which were the most abundant and second most abundant clonotypes among TIL and PB-post T cells, were not the most prominent clones among PB-pre T cells. This seemed to confirm the above-stated suspicion, based on UMAP proximity, that PB-post T cells were shaped by the TIL infusion product, and PB-pre T cells formed a rather distinct subset. Analyzing the phenotypes of clonotypes in each sample confirmed our previous observations from the mouse model that T cells showed cellular states characterized by a 'dysfunctional' signature among TIL and by a 'cytotoxic' signature with high 'activation' scores among PB-pre T cells, but it also revealed highly variable phenotypes depending on the clonotype (Fig. 6f). Importantly, the PB-post sample is expected to be phenotypically biased by T cells that were contained in the infusion product. In contrast, the PB-pre sample should not affected by this. Our preclinical studies indicated that PD-1 protein expression (as a marker for recent T cell activation) could serve as a biomarker of highly functional TCRs in peripheral blood. However, in previous analyses of tumor-specific T cells, *PDCD1* RNA levels seemed to be disconnected from PD-1 protein levels₂₀. In line with this, the inventors saw that *PDCD1* was the only gene of the dysfunctional signature that did not show highest expression in TIL. It was therefore questionable whether *PDCD1* RNA levels would serve as a good biomarker for TCRs with high functionality. Following the notion that PD-1 protein level is indicative of recent cellular activation (rather than having a functional importance as a single molecule), the inventors instead hypothesized that high-functionality TCRs could be best identified in peripheral blood by high activation and cytotoxicity gene scores. In order to test this hypothesis, the inventors engineered primary human T cells to transgenically express selected TCRs from our analysis, using CRISPR/Cas9-mediated orthotopic TCR replacement (OTR) (Schober, K. et al. Orthotopic replacement of T-cell receptor α- and β-chains with preservation of near-physiological T-cell function. Nat. Biomed. Eng. 3, 974-984 (2019)). This technology places the transgenic TCR into the endogenous gene locus, resulting in physiological transgene expression, and simultaneously completely removes the endogenous TCR, leading to unbiased and complete re-expression of transgenic TCRs. Because the recipient T cells for engineering are the same for all TCRs, this approach allows a systematic comparison of TCR functionality, irrespective of the phenotype of the original cells from which the TCR was isolated. For re-expression the inventors chose the 8 most abundant TCRs across all samples (TCRs 14, 2, 4, 5, 23, 49, 13; 74). In addition, the inventors selected TCR 184, which was disproportionately abundant among PB-post T cells, and TCR 471, which showed the highest activation score among PB-pre T cells, but was not found among TIL or PB-post T cells (Fig. 6f). After transgenic re-expression by OTR, all TCRs except for TCR 184 showed a positive, transgenic TCR-dependent ENTPD4_{P>L} pMHC multimer staining (Fig. 6g). To test antigen specific functionality, the inventors co-incubated engineered T cells with HLA-A*01:01-expressing K562 cells, loaded with ENTPD4_{P>L} antigen, and analyzed release of IFNγ, TNFα and IL-2. This confirmed antigen-specific functionality for all TCRs except for TCR 184 (Fig. 6h). Functional avidity is best reflected by peptide sensitivity. TCR 4 showed the highest peptide sensitivity (Fig. 7i). These data showed that the inventors could identify functional TCRs with differential peptide sensitivity. Finally, the inventors aimed to test whether TCR-intrinsic functionality could be predicted by phenotypic signatures. To this end, the inventors probed correlations of functional avidity against clonotype abundance and phenotype in the different samples, with a particular focus on PB-pre T cells (Fig. 6j), since this represents a widely accessible source of TCRs in tumor patients before immunotherapy. Also, as noted above, the TIL infusion product and the PB-post T cells are expected to be phenotypically skewed by the *ex vivo* T cell expansion of the TIL infusion product before therapeutic application. Functional avidity correlated only poorly with clonotype abundance ('size') and, as expected, with *PDCD1* expression levels and the frequency of cells with detectable *PDCD1* transcript in PB-pre T cells (Fix. 6j). Also, neither the 'predysfunctionality' nor the 'dysfunctionality' score of PB-pre T cells robustly correlated with TCR functionality. The 'activation' and 'cytotoxicity' scores, in contrast, correlated robustly with TCR functionality across all measured cytokines in PB-pre T cells, but not in TIL (Fig. 6jk). These analyses confirmed that high-functionality TCRs can be identified by high 'cytotoxicity' or 'activation' signatures in peripheral blood. the inventors wondered which individual genes would form the best biomarkers for the TCRs with different functionality categories. To this end, the inventors calculated how well individual gene expression of a given clonotype *in vivo* among PB-pre T cells correlated with peptide sensitivity of the same clonotype's TCR after transgenic re-expression (Fig. 6l-m). This analysis identified *GZMB* and CCL3 as genes from the 'activation' signature that could be found among genes that correlated strongly and statistically significantly with TCR functionality. One of the top hits further included *UNC13D*, which has been described to be important for the cytotoxic activity of T cells. Interestingly, the most strongly correlating genes were also enriched for genes associated with T cell survival, including (but not restricted to) T cell quiescence regulating *BTG1* and *BTG2*; the molecular chaperone Clusterin encoding CLU₆₇; the insulin receptor substrate (IRS) 1 encoding *IRS1* (which protects T cells from activation-induced cell death); or the antiapoptotic Bcl-2 family member *BCL2L2.* Overall, the inventors could identify individual genes in human tumor-specific T cell clones from peripheral blood that can serve as biomarkers to identify high-functionality TCRs.

### MATERIALS AND METHODS

### REAGENT or RESOURCE SOURCE IDENTIFIER

**Table 1**

| **Antibodies** |
|---|
| **murine** |
| aCD45.1 FITC BioLegend 110706 |
| aCD45.1 PE BioLegend 110708 |
| aCD45.1 PerCP-Cy5.5 BioLegend 110728 |
| aCD45.2 PerCP-Cy5.5 BioLegend 109828 |
| aCD45.2 Pacific Blue BioLegend 109820 |
| aCD90.1 APC Life Technologies 17-0900-82 |
| aCD90.1 FITC Life Technologies 11-0900-85 |
| aCD90.2 APC-eF780 Invitrogen 47-0902-80 |
| aCD90.1 PE Life Technologies 12-0900-83 |
| aCD27 PE Invitrogen 12-0271-83 |
| aCD62L APC Biolegend 104412 |
| aCD62L PE-Cy7 BioLegend 104418 |
| aCD19 PE-CF594 BD 562291 |
| aCD8a Pacific Blue Invitrogen MCD0828 |
| aCD8a Pacific Blue Biolegend 100725 |
| aCD8a PE-Cy7 BioLegend 100722 |
| aCD8a Pacific Orange LifeTechnologies MCD0830 |
| aCD44 FITC Biolegend 103022 |
| aPD-1 FITC Life Technologies 11-9985-81 |
| aIFNγ APC Invitrogen 17-7311-82 |
| aTNFa PE-Cy7 BD 557644 |
| aIL-2 PE Life Technologies 12-7021-82 |
| aSca1 PE-Cy7 Life Technologies 25-5981-81 |
| aTIM-3 PE-Cy BioLegend 134009 |
| aLAG-3 APC Life Technologies 17-2231-80 |

### Bacterial Strains

- Listeria monocytogenes-Ovalbumin MIH Munich

### Chemicals, Peptides, Enzymes

- Propidium Iodide (PI) Life Technologies Cat#P1304MP
- Ethidium Monoazide Bromide (EMA) Life Technologies Cat#E1374
- Percoll GE Healthcare 17-0891-01
- Deoxyribonuclease I from bovine pancreas Sigma Aldrich D4513
- Collagenase from Clostridium histolyticum Sigma Aldrich C2139

### Experimental Models: Cell Lines

- Platinum E cells Cell Biolabs Cat#RV-101
- MC38OVA Kind gift of Wolfgang Uckert
- PancOVA Kind gift of Max Schnurr

### Experimental Models: Organisms/Strains

- C57BI/6JOIaHsd Envigo Stock number: 057

### MHC multimer

- H2-Kb/mβ2m/Ova 257-264 Streptavidin-PE/APC

### Software and Algorithms

- FloJo V10 FlowJo LLC
- Prism 8 Graphpad
- MetaMorph Online software Molecular Devices
- Microsoft Excel Microsoft
- RTCA Software 2.0 Acea Bioscience, Inc.

### Mice

C57BI/6JOIaHsd (females, 6-8 weeks) were purchased from Envigo. 6 - 24 weeks old female SIINFEKL peptide-specific TCR transgenic OT-I (C57BI/6-Tg(TcraTcrb)1100Mjb/J) were originally obtained from The Jackson Laboratory and bred under specific pathogen-free conditions at our mouse facility at the Technical University of Munich. Different congenic marker backgrounds of CD45.1/.2 and CD90.1/.2 were derived from in-house breeding39. All animal experiments were approved by the district government of upper Bavaria (Department 5 - Environment, Health and Consumer Protection).

**Human blood samples**. Written informed consent was obtained from the donors and use of the blood samples was approved according to national law by the Dutch (CCMO) or by the German (Ethikkommission der Medizinischen Fakultät der Technischen Universität München) Ethics Institutional Review Board, according with the Declaration of Helsinki and Good Clinical Practice. Further clinical information on the human melanoma patient has been previously described (van den Berg, J. H. et al. Tumor infiltrating lymphocytes (TIL) therapy in metastatic melanoma: boosting of neoantigen-specific T cell reactivity and long-term follow-up. J. Immunother. Cancer 8, e000848 (2020). Listeria Infection with recombinant Listeria monocytogenes expressing Ovalbumin was performed by i.v. injection of 5000 CFU81. Tumor model Mice were injected subcutaneously in the right flank with 106 tumor cells. Tumors were measured every 2-3 days with a caliper. Tumor size was determined as product of two diameters (length × width). Upon reaching 225mm2 or ulceration of the tumor, mice were sacrificed. For in vivo transfer experiments, mice were irradiated with 5 Gy followed by intraperitoneal injection of T cells. Where indicated, tumors were excised, mechanically disaggregated and enzymatically digested with collagenase type VIII (125 CDU/mg, Sigma- Aldrich) and DNAse I (2000 U/mg, Sigma Aldrich) for 60 minutes at 37°C. Tumor cell suspensions were passed through a 70-µm cell strainer and lymphocytes were enriched in a Percoll (GE Healthcare) density gradient (40% vs. 80%) at 3600g for 20 minutes. Generation of T cell retrogenic mice Retrogenic mice were generated as described before37. In brief, bone marrow was harvested from congenically marked donor mice with a Rag-1-/- background. Sca-1 positive HSCs were retrovirally transduced with a given TCR and injected i.v. into irradiated C57BL/6 mice.

**Flow cytometry.** Tumor cell suspensions were produced as previously described. Axillary and inguinal lymph nodes were harvested and mashed through a 40-µm cell strainer to generate a single cell suspension. Blood was obtained bleeding from the facial vein. Spleens were harvested and mashed through 70-µm cell strainers. Following red blood cell lysis with Tris-Buffered Ammonium Chloride, cells were stained with the respective antibody panel for 30 min at 4 °C in the dark (for multimer staining see below). After washing with FACS buffer (PBS with 0,5% BSA and 2 mM EDTA), the cells were stained with PI for 5 min and washed again. Data were collected by flow cytometry on a CyAn ADP 9 color (Beckman Coulter) or Cytoflex S flow cytometer (Beckman Coulter). For analysis, FlowJo software (FlowJo LLC) was used.

**Adoptive transfer and tracking of T cells from TCR retrogenic mice.** For adoptive transfer experiments TCR retrogenic donors with TCRs of different affinities for H2Kb/SIINFEKL on a unique congenic marker background (CD45, CD90) were used. Blood from retrogenic mice was stained following red blood cell lysis with antibodies directed against CD8 and CD44, with PI staining for live/dead discrimination. Naïve T cells (living, CD8+, CD44_{low}, congenic₊) were sorted (MoFlo legacy; Beckman Coulter) into a 96-well v-bottom plate containing a cell pellet of 400.000 C57BI/6 splenocytes in FCS. Cells were injected i.p. into WT recipients. Peripheral blood of recipient mice was analyzed via flow cytometry at different timepoints after transfer. The unique congenic marker backgrounds allowed tracking of individual TCRs over time.

**Peptide-MHC (pMHC) multimer staining.** Biotinylated pMHC molecules for the generation of pMHC multimers were refolded according to the protocol described in₈₂. 0,4 µg of biotinylated pMHC I molecule, 0,5 µg of Streptavidin- PE or Streptavidin-APC and 50µl of FACS buffer for every 5×10₆ cells, were preincubated for at least 30 min for multimerization. Cells were then incubated with the multimer mix for 30 min. 20 min before the end of the staining period antibodies for the staining of surface antigens were added. PI for live/dead staining was added 5 min before the end of the staining period.

**Peptide titration and intracellular cytokine staining.** Samples were split and incubated with different peptide concentrations (10₋₆-10₋₁₂ M) of the SIINFEKL-peptide, a negative (RP10₊ only) and a positive control (PMA/lonomycin). Samples were then incubated at 37 °C for five hours, after one hour 2 µg/well of Golgi Plug was added without resuspension. Next EMA/Fc-block staining (EMA 1:1000, Fc-Block 1:400) and staining of surface antigens (CD8, CD19, CD45.1/CD90.1) were performed. After fixation and permeabilization using Cytofix/Cytoperm (BD) intracellular cytokine staining (IFN*γγ*, TNF*αα*, IL2) was performed. The maximal percentage of IFNγ+ CD8+ cells was normalized to 100%, and a nonlinear curve was fitted into the normalized data.

***In vivo L.m.* OVA proliferation.** Mice were injected with 100 retrogenic T cells for each TCR and infected with 5000 CFU *L.m.* OVA (both i.v.). On day 12 after infection, spleens were harvested, processed as described above and subsequently analyzed by flow cytometry.

**Analysis of *in vitro* cytotoxicity.** Experiments were performed using the xCELLigence technology (Roche/ACEA Biosciences, San Diego, CA) at 37°C with 5% CO₂. For measuring cytotoxic responses, 10₄ PancOVA cells were seeded on 96 well E-Plates (E-Plate^{®} 96) with gold micro electrodes. Changes in impedance were monitored at 15-minute intervals for up to 120 hours. After 24 hours the supernatant was removed and T cells were added to the culture. All incubations were performed in volumes of 200 ul of cDMEM. Cell index (CI) values were analyzed by the RTCA Software 2.0 (Acea Bioscience, Inc.). For time point-independent and thus more unbiased analysis, the area-under-the-curve of cell index values was determined to indicate killing capacity.

**Tissue culture.** All cell lines were grown in cDMEM (DMEM (Life Technologies), supplemented with 10% FCS, 0.025% L-Glutamine, 0.1% HEPES, 0.001% gentamycin and 0.002% streptomycin).

Single cell RNA sequencing. After cells have been sorted, they were centrifuged and the supernatant was carefully removed. Cells were resuspended in the Mastermix + 37.8 µl of water before 70 µl of the cell suspension were transferred to the chip. (Step 1.1 and 1.2 of the original protocol). After each step, the integrity of the pellet was checked under the microscope to ensure that all cells are loaded onto the chip. From here on, 10x experiments have been performed according to the manufacturer's protocol (Chromium next GEM Single Cell VDJ V1.1 with Feature Barcode, Rev D). QC has been performed with a High sensitivity DNA Kit (Agilent #5067-4626) on a Bioanalyzer 2100 as recommended in the protocol and libraries were quantified with the Qubit dsDNA hs assay kit (life technologies #Q32851). All steps have been performed using RPT filter tips (Starlab #S1183-1710, #SS1180-8710, #S1182-1730) and DNA LoBind tubes (Sigma #EP0030108051, #EP0030108078, #EP0030124359).

**scRNA seq data analysis.** References GRCh38-2020-A and vdj_GRCh38_alts_ensembl-5.0.0 were used for Transcriptome and VDJ annotation (CellRanger 5.0.0), respectively. Data analysis was performed with SCANPY (V1.4.3, Wolf et al 2018). The notebooks containing all steps of data processing and analysis can be found online (GitHub repository)

**TCR DNA template design.** DNA templates were designed in silico and synthesized by Twist Bioscience. DNA constructs for CRISPR/Cas9-mediated homology-directed repair (HDR) had the following structure: 5' homology arm (300-400 bp), P2A, TCR β (including murine TRBC with additional cysteine bridge (Cohen CJ, et al 2006, Cohen CJ, et al 2007, Kuball J et al. 2007)), T2A, TCR α (including murine TRAC with additional cysteine bridge (Cohen CJ, et al 2006, Cohen CJ, et al 2007, Kuball J et al. 2007)), bGHpA tail, 3' homology arm (300-400 bp). The homology arm sequences of the TRBC locus were derived from TRBC1 and are highly homologous to TRBC2.

**CRISPR/Cas9-mediated KO and KI**. Cas9 RNPs and HDR repair templates were generated as described before. Bulk PBMCs were activated for two days in RPMI with CD3/CD28 Expamer (Juno Therapeutics), 300 IU ml₋₁ IL-2, 5 ng ml₋₁ IL-7 and 5 ng ml₋₁ IL-15. Expamer stimulus was removed by incubation with 1 mM D-biotin. Cells were electroporated (pulse code EH100) with Cas9 ribonucleoprotein and DNA templates in Nucleofector Solution (20 µl per 1 × 106 T cells; Lonza) with a 4D Nucleofector X unit (Lonza). After electroporation, cells were cultured in RPMI with 180 IU ml-1 IL-2 until a first FACS analysis on day five after editing.

**Antigen-specific activation and intracellular cytokine staining**. On the day before co-culture with T cells, K562 cells (retrovirally transduced to express the human MHC class-I molecule of interest) were irradiated (80 Gy) and loaded with peptide (10_{- 12} M, 10₋₁₀ M, 10₋₉ M, 10₋₈ M, 10₋₇ M, 10₋₆ M, 10₋₅ M, 10₋₄ M) overnight at 37°C. T cells were cocultured with peptide-loaded K562 cells and Golgi plug (BD Biosciences) in a 1:1 ratio for 4 h at 37°C. PMA (25 ng ml-1) and ionomycin (1µg ml-1) were used for positive control. Surface marker antibody staining for CD3 (BV421, BD Biosciences), CD8 (PE, Invitrogen) and antimurine TCR β-chain (APC/Fire750, BioLegend) was followed by permeabilization using Cytofix/Cytoperm (BD Biosciences), and intracellular staining of IFNγ (FITC, BD Pharmingen), TNFα (PC7, eBioscience) and IL-2 (APC, BD Biosciences). Live/dead discrimination was performed with ethidium-monoazide-bromide (Invitrogen).

**Statistical analyses**. Statistical analyses were performed using the GraphPad PRISM software. Statistical tests were used as indicated.

### REFERENCES

Alanio, C., Lemaitre, F., Law, H. K. W., Hasan, M. & Albert, M. L. Enumeration of human antigen-specific naive CD8+ T cells reveals conserved precursor frequencies. Blood 115, 3718-25 (2010).

Becht, E. et al. Dimensionality reduction for visualizing single-cell data using UMAP. Nat. Biotechnol. 37, 38-44 (2019).

Bos, R., Marquardt, K. L., Cheung, J. & Sherman, L. A. Functional differences between low- and high-affinity CD8 + T cells in the tumor environment. Oncoimmunology 1, 1239-1247 (2014).

Buchholz, V. R. et al. Disparate individual fates compose robust CD8+ T cell immunity. Science 340, 630-5 (2013).

Caserta, S., Kleczkowska, J., Mondino, A. & Zamoyska, R. Reduced Functional Avidity Promotes Central and Effector Memory CD4 T Cell Responses to Tumor- Associated Antigens. J. Immunol. 185, 6545-6554 (2010).

Enouz, S. et al. Autoreactive T cells bypass negative selection and respond to selfantigen stimulation during infection. J Exp Med 209, 1769-1779 (2012).

Gros, A. et al. PD-1 identifies the patient-specific CD8+ tumor-reactive repertoire infiltrating human tumors. J. Clin. Invest. 124, 2246-59 (2014).

Gros, A. et al. Prospective identification of neoantigen-specific lymphocytes in the peripheral blood of melanoma patients. Nat. Med. (2016) doi:10.1038/nm.4051

Grassmann, S. et al. Distinct Surface Expression of Activating Receptor Ly49H Drives Differential Expansion of NK Cell Clones upon Murine Cytomegalovirus Infection. Immunity 1-10 (2019) doi:10.1016/j.immuni.

Martínez-Usatorre, A., Donda, A., Zehn, D. & Romero, P. PD-1 Blockade Unleashes Effector Potential of Both High- and Low-Affinity Tumor-Infiltrating T Cells. J. Immunol. ji1701644 (2018) doi:10.4049/jimmunol.1701644.

Schober, K. et al. Orthotopic replacement of T-cell receptor α- and β-chains with preservation of near-physiological T-cell function. Nat. Biomed. Eng. 3, 974-984 (2019).

Schober, K. et al. Reverse TCR repertoire evolution toward dominant low-affinity clones during chronic CMV infection. Nat. Immunol. 21, 434-441 (2020).

Stadtmauer, E. A. et al. Long-term safety and activity of NY-ESO-1 SPEAR T cells after autologous stem cell transplant for myeloma. Blood Adv. 3, 2022-2034 (2019).

Sims, J. S. et al. Diversity and divergence of the glioma-infiltrating T-cell receptor repertoire. Proc. Natl. Acad. Sci. 201601012 (2016) doi:10.1073/pnas.1601012113).

Thommen, D. D. et al. A transcriptionally and functionally distinct PD-1 + CD8 + T cell pool with predictive potential in non-small cell lung cancer treated with PD-1 blockade. Nat. Med. (2018) doi:10.1038/s41591-018-0057-z.).

Traag, V. A., Waltman, L. & van Eck, N. J. From Louvain to Leiden: guaranteeing well connected communities. Sci. Rep. 9, 5233 (2019).

Tscharke, D. C., Croft, N. P., Doherty, P. C. & La Gruta, N. L. Sizing up the key determinants of the CD8(+) T cell response. Nat. Rev. Immunol. 15, 705-16 (2015).

Van den Berg, J. H. et al. Tumor infiltrating lymphocytes (TIL) therapy in metastatic melanoma: boosting of neoantigen-specific T cell reactivity and long-term follow-up. J. Immunother. Cancer 8, e000848 (2020).

Van der Leun, A. M., Thommen, D. S. & Schumacher, T. N. CD8+ T cell states in human cancer: insights from single-cell analysis. Nat. Rev. Cancer 20, 218-232 (2020).

## Claims

1. A method of identifying one or more T cells comprising:
a. determining in a peripheral blood sample derived from a subject the expression level of PD-1 in a population of T cells; and
b. identifying one or more T cells that have an expression level of PD-1 that is above the median expression of PD-1 in the population of T cells.

2. The method of claim 1 wherein PD-1 expression is PD-1 protein expression.

3. The method of claim 1 or 2, wherein the one or more T cells are identified if the one or more T cells have an expression level of PD-1 that is within about the top 20%, preferably within about the top 10%, within about the top 5%, preferably within about the top 4% of the PD-1 expression level found in said population of T cells.

4. A method of identifying one or more T cells comprising:
a. determining in a peripheral blood sample the mRNA expression level of one or more markers selected from the group consisting of GZMB, CCL3, UNC13D, BTG1, BTG2, CLU, IRS1, and BCL2L in a population of T cells; and
b. identifying one or more T cells that fulfill at least one of the following features:
i. increased GZMB expression;
ii. increased CCL3 expression;
iii. increased UNC13D expression;
iv. increased BTG1 expression;
v. increased BTG2 expression;
vi. increased CLU expression;
vii. increased IRS1 expression;
viii. increased BCL2L expression.

5. The method of any one of the preceding claims, wherein the method is for identifying one or more tumor-antigen specific T cells.

6. A method of isolating one or more T cell, comprising isolating one or more T cells identified according to a method of any one of claims 1-5.

7. A method of identifying one or more T cell receptors (TCR) comprising determining the sequence of the TCR of the one or more T cells identified by the method of any one of claims 1-5.

8. A nucleic acid molecule comprising a nucleic acid sequence encoding the TCR identified by the method of claim 7, or encoding a TCR comprised in a T cell identified by the method of any one of claims 1-5.

9. A method of generating one or more immune cells comprising introducing one or more nucleic acid molecules encoding one or more TCRs or antigen-binding fragments thereof, identified by the method of claim 7 into one or more host cells.

10. The method of claim 9, wherein the host cell is an immune cell.

11. A host cell comprising a nucleic acid molecule of claim 8 or generated by the method of any claim 9 or 10.

12. A pharmaceutical composition comprising a T cell identified by the method of any one of claims 1-5 or isolated by the method of claim 6, or an immune cell generated by the method of claim 9 or 10, or a host cell of claim 11.

13. A T cell identified by the method of any one of claims 1-5 or isolated by the method of claim 6, or an immune cell generated by the method of claim 9 or 10, or a host cell of claim 11, for use in therapy.

14. The T cell, immune cell, or host cell for the use of claim 13, wherein the therapy is adoptive T cell therapy.

15. A method of diagnosing cancer in a subject comprising measuring the PD-1 expression level in a peripheral blood sample obtained from the subject, wherein an elevated PD-1 expression level as compared to a reference peripheral blood sample from a healthy control indicates the presence of T cells having high affinity TCRs.
